# EUROPÄISCHE PATENTSCHRIFT

(11) **EP 3 192 499 B1**
(45) Veröffentlichungstag und Bekanntmachung des Hinweises auf die Patenterteilung: **29.11.2023**
(21) Anmeldenummer: 17000074.9
(22) Anmeldetag: 13.01.2017
(51) Int. Cl.: A61K 9/00, A61K 47/38, A61K 9/70

(54) **CELLULOSEFASERBASIERTE TRÄGERMATRICES FÜR SCHICHTARTIGE PRODUKTE ZUR ORALEN UND PERORALEN APPLIKATION SOWIE DEREN HERSTELLUNG**
CELLULOSE FIBRE BASED SUPPORT MATRICES FOR LAYERED PRODUCTS FOR ORAL AND PERORAL APPLICATION AND THEIR PREPARATION
MATRICE DE SUPPORT À BASE DE FIBRES DE CELLULOSE POUR DES PRODUITS STRATIFIÉS DESTINÉS À L'APPLICATION ORALE OU PÉRORALE ET SA FABRICATION

(30) Priorität: 14.01.2016 DE 102016000541
(43) Veröffentlichungstag der Anmeldung: 19.07.2017
(73) Patentinhaber: Glatt GmbH, 79589 Binzen (DE)
(72) Erfinder: LEMKE, Stefan, 12203 Berlin (DE); STRÄTLING, Ernst-Josef, 98704 Wolfsberg (DE); WELZEL, Hans-Peter, 14547 Beelitz OT Busendorf (DE); KECK, Cornelia Margarete, 14548 Schwielowsee (DE)
(74) Vertreter: Breuer, Markus

(56) Entgegenhaltungen:
- DE-A1- 3 344 772
- US-B2- 6 884 790
- GERNOT JÃ GER ET AL: "How recombinant swollenin from Kluyveromyces lactis affects cellulosic substrates and accelerates their hydrolysis", BIOTECHNOLOGY FOR BIOFUELS, BIOMED CENTRAL LTD, GB, Bd. 4, Nr. 1, 23. September 2011 (2011-09-23), Seite 33, XP021111197, ISSN: 1754-6834, DOI: 10.1186/1754-6834-4-33

## Beschreibung

### Technisches Gebiet

Die Erfindung betrifft cellulosefaserbasierte Trägermatrices auf Papierbasis (smartFilms) zur Herstellung schichtartiger Produkte zur oralen und peroralen Applikation sowie deren Herstellungsverfahren.

Die orale Applikation betrifft dabei die Verabreichung der Darreichungsform im Mundraum beispielsweise sublingual oder bukkal, wobei die Inhaltsstoffe insbesondere der oder die Wirkstoff(e) nach Freisetzung aus dem smartFilm lokal und/oder systemisch wirken können.

Die perorale Applikation betrifft dabei die Verabreichung der Darreichungsform über den Mund beispielsweise gastrointestinal, wobei die Inhaltsstoffe insbesondere der oder die Wirkstoff(e) nach Freisetzung aus dem smartFilm lokal und/oder systemisch wirken können.

### Stand der Technik

Orale Filme auch als orale Streifen, Scheiben oder Pflaster bezeichnet, sind kommerziell im Markt bekannt seit den frühen 2000er Jahren, durch die Einführung des Mundhygieneproduktes Listerine^{®} Pocketpacks^{®} Breath Strips zur Atemerfrischung. Nach dieser Einführung sind verschiedene orale Filmprodukte für verschiedenste Anwendungsmöglichkeiten entwickelt und vermarktet worden. So folgten beispielsweise Crest^{®} 3D Whitestrips^{®} (Wirkstoff: Wasserstoffperoxid) als Kosmetikum zum Weißen der Zähne zur dentalen Applikation, Sheets^{®} Energy (Wirkstoff: Koffein) als Nahrungsergänzungsmittel zur peroralen Applikation mit gastrointestinaler Resorption und Suboxone^{®} Film (Wirkstoff: Buprenorphinhydrochlorid, Naloxonhydrochlorid) als Arzneimittel zur sublingualen oder bukkalen Applikation.

Konventionell verfügbare orale Filme zeichnen sich durch eine dünne, in den meisten Fällen rechteckige Form aus, vergleichbar der Gestalt einer Briefmarke. Im Vergleich zu anderen häufig eingesetzten oralen Applikationsformen mit einer klaren dreidimensionalen geometrischen Gestalt wie z.B. Tabletten oder Kapseln sind orale Filme eher ebene Folien aus einer oder mehreren Schichten, welche allerdings auch geknickt, gerollt oder geknüllt eingenommen werden können.

Orale Filme sind eine feste aber ziemlich flexible orale Applikationsformen, wohingegen andere häufig genutzte feste Oralia wie z.B. Tabletten eher spröde und zerbrechlich sind. Dies erleichtert die Anwenderhandhabung, aber auch die Verpackung, Lagerung und den Transport der oralen Filme.

Im Allgemeinen bilden ein oder mehrere Polymere die Trägergrundlage von oralen Filmen. Derzeit verwendete Beispiele für Polymere sind modifizierte Celluloseether (z. B. Hydroxypropylmethylcellulose), Gelatine, Pullulan, Pektin oder Stärke.

Ein oder mehrere Weichmacher dienen dazu, durch Erniedrigung der Glasübergangstemperatur des Polymers, die Flexibilität des oralen Films zu erhöhen und die Sprödigkeit zu senken. Beispiele für derzeit in oralen Filmen genutzte Weichmacher sind Glycerol, Propylenglykol, Triethylcitrat, Glyceroltricitrat und Polyethylenglykolderivate.

Die Formulierung der oralen Filme kann, neben den obligatorischen Filmformungshilfsstoffen (Polymer(e) und Weichmacher), durch fakultative Inhaltsstoffe wie Süßungsmittel z.B. Glucose, Geschmacksmaskierer (z.B. ätherische Öle), Farbstoffe (z.B. Brilliantblau) oder weiteren Hilfsstoffen vervollständigt werden.

Orale Filme werden konventionell hergestellt durch das Lösungsmittelgießverfahren (engl. solvent-casting) oder durch das Schmelzextrusionsverfahren (engl. hot melt extrusion) mit unzählig verschiedenen Methodenparametern und Materialien.

Nachteilig ist, dass derzeit die Beladungskapazität mit Inhaltsstoffen insbesondere Wirkstoffen in oralen Filmen begrenzt ist durch deren Größe und Applizierbarkeit, weswegen bisher nur hochaktive Wirkstoffe in geringen Dosen verwendet werden können.

In der Veröffentlichung US 20030206942 A1 wird beschrieben, dass die relative Wirkstoffbeladung bezogen auf die Gesamtmasse vorzugsweise zwischen 5 und 30 % (m/m) schwankt. Neben diesem limitierten relativen Wirkstoffgehalt des eigentlichen für die Wirkung verantwortlichen aktiven Wirkstoffes erfolgt der Einsatz weiterer Inhaltsstoffe. So wurde in dieser Veröffentlichung auch beschrieben, dass zur Filmformung nicht nur das obligatorisch erforderliche Polymer als strukturgebende Komponente zum Einsatz kommt, sondern auch für die Wirkung nicht erforderliche Hilfsstoffe wie Weichmacher, Stabilisatoren und Verdickungsmittel. Die derzeitig erreichte relative Beladung zwischen 5 und 30 % (m/m) bezogen auf die Gesamtmasse ist für eine breite Auswahl möglicher Wirkstoffkandidaten unbefriedigend. Dies limitiert die Applikationsform der oralen Filme auf wenige ausgewählte Wirkstoffe. So entsprechen bei einem Gesamtgewicht des oralen Films von beispielsweise 50 mg nur maximal 15 mg der Beladung mit Wirkstoff.

In US 6 884 790 B2 werden ein Filterpapier (Type 520 8 von Schleicher & Schüll) und eine Filtertuch aus Polypropylen verwendet als Trägermatrizen. Auf diese Träger wurde eine Cyclodextrin-Lösung aufgetragen.

Bekannt ist aus der Veröffentlichung CA 2800968 A1 das zur Filmformung inklusive Weichmacher mindestens fünf verschiedene Hilfsstoffe eingesetzt werden.

Des Weiteren ist davon auszugehen, dass die in der Veröffentlichung US 20030206942 A1 beschriebenen Beispiele oraler Filme, aufgrund des hydrophilen Charakters verschiedener Inhaltsstoffe insbesondere des strukturgebenden Polymers einen unbestimmten Anteil an Wasser mit inkorporiert und/oder adsorbiert haben. Dies stellt insbesondere für feuchtigkeitslabile Inhaltstoffe ein Instabilitätsrisiko dar.

Sowohl beim Lösungsmittelgießverfahren, wie beschrieben in der Veröffentlichung US 0039932 A1, als auch beim Schmelzextrusionsverfahren, wie beschrieben in der Veröffentlichung EP 0250187 A2, erfolgt der Filmformungsprozess in Anwesenheit aller Inhaltsstoffe insbesondere dem oder der Wirkstoff(e).

Nachteilig an beiden Verfahren ist, dass der Filmformungsprozess gestört werden kann beispielsweise durch einen Antiweichmachereffekt verursacht durch weitere Inhaltsstoffe wie Hilfsstoffe und/oder Wirkstoffe.

Auch erfahren die Inhaltsstoffe insbesondere der oder die Wirkstoff(e) durch diese Vorgehensweise zusätzlichen thermischen Stress während des Filmformungsprozess beispielsweise beim Trocknen oder beim Extrudieren. So erfährt die Filmmischung bestehend aus Hilfstoffen und Wirkstoffen der Veröffentlichung EP 0250187 A2 verschiedene Temperaturen sogar bis zu 185 °C. Dieser thermische Stress ist für verschiedenste Inhaltsstoffe insbesondere dem oder der Wirkstoff(e) nicht akzeptierbar.

Nachteilig ist neben der geringen relativen Beladung an Wirkstoffen auch, dass die Abhängigkeit des Filmformungsschrittes vom Beladungsschritt dazu führt, dass der orale Film nach Herstellung nicht oder nur begrenzt weiter modifiziert werden kann. Jede anwenderindividuelle Modifizierung erfordert in der Regel die Herstellung eines neuen oralen Films mit einer neuen angepassten Rezeptur. So stehen derzeit nur sehr spezielle unflexible Herstellungsmethoden zur Verfügung, welche nicht für eine individuelle Anwendung beispielsweise eine personalisierte Therapie geeignet sind.

Bevorzugt ist das amorphe (griechisch für ohne Gestalt) Vorliegen der Inhaltsstoffe insbesondere des oder der Wirkstoff(e) gegenüber dem kristallinen Zustand in der Applikationsform. Der amorphe Zustand besitzt genau wie der kristalline Zustand dieselbe Nahordnung allerdings keine Fernodnung wie dies im kristallinen Zustand der Fall ist. Die einzelnen Moleküle sind unregelmäßig und nicht geordnet verknüpft. Sie besitzen keine explizite geometrische Anordnung. Der Vorteil zwischen amorphen Strukturen gegenüber kristallinen Strukturen ist, dass weniger Energie aufgebracht werden muss, um diese zu lösen. Dies führt zu einer Erhöhung der Lösungsgeschwindigkeit, denn amorphe Substanzen lösen sich schneller auf, da beim Lösungsprozess keine Gitterenergie wie bei kristallinen Strukturen überwunden werden muss.

Insbesondere haben amorphe Inhaltsstoffe insbesondere Wirkstoffe im Vergleich zu kristallinen eine höhere Sättigungslöslichkeit cₛ, was zu einem erhöhten Konzentrationsgradienten c_{Donor}-c_{Akzeptor} zwischen Donorseite (z. B. Flüssigkeit in der Mundhöhle) und Akzeptorseite (z. B. Zellen, Blut) führt, nachfolgend zu erhöhtem diffusivem Fluss ins Blut und somit zu einer erhöhten Bioverfügbarkeit.

Problematisch ist jedoch die physikalische Instabilität des amorphen Zustandes der Inhaltsstoffe insbesondere der Wirkstoffe, da diese tendieren zu rekristallisieren. Rekristallisation von amorphen oder gelösten Wirkstoffen in polymeren filmartigen Applikationsformen beispielsweise in transdermalen Pflastern (engl. patches) ist ein bekanntes Problem, welches auch in oralen Filmen auftritt. Die Verbindung von erhöhter Lösungsgeschwindigkeit dc/dt mit erhöhter Sättigunsglöslichkeit cₛ führt neben einer erhöhten Resorption auch zu einer sehr schnellen Resorption, wie sie oft in der Mundhöhle erwünscht ist.

Erhöhte Sättigungslöslichkeit cₛ und erhöhte Lösungsgeschwindigkeit dc/dt kann auch mit Nanokristallen erreicht werden. Nanokristalle sind hochdisperse Systeme. Hochdisperse Systeme sind a priori instabil, das heißt sie neigen zur Aggregation oder Agglomeration. Dadurch verlieren diese ihre speziellen Nanoeigenschaften. Die Herausforderung beim Einarbeiten in Filmen besteht darin, ihre Integrität weitestgehend zu erhalten, erkennbar daran, dass sie auch aus Filmen eine erhöhte cₛ und dc/dt besitzen. Nanokristalle in smartFilms, wie in der vorliegenden Erfindung verwendet, wurden bisher nicht beschrieben.

Reine Inhaltsstoffe insbesondere Wirkstoffe aufgebracht auf smartFilms in Form von Kristallen, Nanokristallen oder im amorphen Zustand lösen sich mit einer Geschwindigkeit auf, die bestimmt wird durch physikalische Parameter wie Oberfläche und Lösungsdruck. Die Möglichkeit zur Modulation der Auflösung und damit kontrollierten Freisetzung von auf smartFilms aufgebrachten Inhaltsstoffen insbesondere Wirkstoffen ist begrenzt z. B. prolongiert (engl. prolonged) oder anhaltend (engl. sustained). Zur Erzielung einer kontrollierten Freisetzung muss der Inhaltsstoff insbesondere Wirkstoff in einer Partikelmatrix eingebracht sein, z. B. eingearbeitet in organische Polymermikro- oder nanopartikel, Lipidmikro- oder nanopartikel oder Partikel aus anorganischen Materialien wie z. B. Silica. Einarbeitung in eine Matrix hat zusätzlich den Vorteil, dass chemisch labile Inhaltsstoffe insbesondere Wirkstoffe im Innern der Matrixpartikel gegen äußere Einflüsse besser geschützt sind.

Der Erfindung liegt die Aufgabe zugrunde, 1.) eine geeigneten smartFilm zu finden und 2.) einen

Herstellungsprozess für Filmprodukte zur oralen und peroralen Applikation zu entwickeln, dadurch charakterisiert, dass
a) eine Aufbringung von Inhaltsstoffen insbesondere Wirkstoffen unabhängig vom Filmformungsprozess möglich ist, d. h. nachträgliche Beladung nach Formung des Films,
b) die Dosis pro Film simpel variiert werden kann, im Gegensatz zum bisher gängigen Lösungsmittelgießverfahren oder Schmelzextrusionsverfahren mit vorgegebener starrer Rezepturzusammensetzung,
c) eine sehr variable Beladung möglich ist, d. h. von sehr geringer Beladung mit hoher Dosiergenauigkeit (z. B. 0,001%; m/m) bis zur deutlich höheren relativen Beladung als in der Literatur beschriebenen mit bis zu 84,2 % (m/m) bezogen auf das Gesamtgewicht der Applikationsform,
d) der Herstellungsprozess industriell kostengünstig realisierbar ist, und
e) das primär verwendete Material für den Film natürlichen und pflanzlichen Ursprungs sowie biologisch abbaubar und somit umweltfreundlich ist (z. B. kein synthetisches Polymer).

In einer besonderen Ausführungsform sollen Filme erzeugt werden mit physikalischen Eigenschaften, die zur Erhöhung der Bioverfügbarkeit der Inhaltsstoffe insbesondere Wirkstoffe führen, d. h. Erhöhung von Lösungsgeschwindigkeit dc/dt und Sättigungslöslichkeit cₛ durch Aufbringen in amorpher Form oder in nanokristalliner Form, wobei der Film diese Eigenschaften konservieren soll, d. h. keine Rekristallisation, keine wesentliche die Lösungsgeschwindigkeit beeinträchtigende Aggregation der Nanokristalle.

In einer weiteren besonderen Ausführungsform sollen die smartFilms mit Matrixpartikeln beladbar sein, wobei die Matrix zur Modulation der Freisetzung und/oder der chemischen Stabilisierung von labilen Wirkstoffen dient.

### Beschreibung

Diese Aufgaben werden erfindungsgemäß gelöst durch die kennzeichnenden Merkmale der Patentansprüche.

Es hat sich gezeigt, dass die Aufgabe durch die erfindungsgemäßen smartFilms als geeignete Grundlage zur Herstellung oraler Filme gelöst wurde.

Papier beschreibt dabei ein flächiges, neben Hilfsstoffen im Wesentlichen aus mechanisch, chemischthermomechanisch oder chemisch aufgeschlossenen Fasern pflanzlicher Herkunft bestehendes Material vorzugsweise Cellulose, welches durch die Entwässerung einer Fasersuspension auf einem Sieb gebildet wird. Dabei entsteht durch Eigenverklebung und Verfilzung von Fasern sowie anschließender Verdichtung und Trocknung ein blattartiges Gebilde, welches als smartFilm zur Herstellung schichtartiger Produkte zur oralen und peroralen Applikation (orale Filme) genutzt wird.

Es hat sich gezeigt, dass smartFilms sich für eine orale Applikation (Verabreichung der Darreichungsform im Mundraum) z. B. buccal oder sublingual sowie perorale Applikation (Verabreichung der Darreichungsform über den Mund) z. B. gastrointestinal mit lokaler und/oder systemischer Wirkung eignen.

Ein weiterer Vorteil ist, dass die erfindungsgemäßen oralen Filme ein kostengünstigeres und verträgliches Alternativsystem mit einem absoluten Minimum an obligatorischen Bestandteilen darstellen, im Vergleich zu bisher verfügbaren oralen Filmen. Überraschenderweise können die erfindungsgemäßen oralen Filme aus smartFilms deutlich höher mit Inhaltsstoffen insbesondere Wirkstoffen beladen werden, kommen ohne Weichmacher aus, sind einfach und leicht industriell adaptierbar herzustellen und können nach Herstellung simpel individuell modifiziert werden. Auch sehr geringe Dosen pro smartFilm können präzise aufgebracht werden. Unter Beibehaltung eines leicht genau zu pipettierenden Volumens werden Lösungen sehr geringer Konzentration aufgebracht (z. B. 50 µl (ca. 50 mg) einer 0,1 %-igen Lösung belädt jeden smartFilm mit konstant 0,05 mg Wirkstoff).

Entgegen dem bisherigen Stand der Technik ist bei der Herstellung der erfindungsgemäßen oralen Filme aus smartFilms der Filmformungsschritt vom Beladungsschritt abgetrennt. Auch die Aufbringung von fakultativen Hilfsstoffen zur Erhöhung der Compliance (Einnahmetreue) des Anwenders ist ohne weiteres möglich. So können beispielsweise Farbstoffe und Aromastoffe für eine Identifizierung des oralen Films sorgen.

Vorteilhaft ist, dass auf smartFilms als Hilfsstoff für orale Filme mit den gewünschten Eigenschaften und benötigten Spezifikationen unproblematisch auch in großen Herstellungsmaßstäben vorgeformt zu beziehen sind. So zeigt sich, dass auch die Struktur des Materials eine entscheidende Rolle für eine bequeme Applikation spielt und eine möglichst hohe Beladungskapazität erlaubt. SmartFilms als stabiles, flexibles und bereits geformtes Material wird mit Wirkstoff(en) und anderen Inhaltsstoffen in Form einer Lösung, Suspension oder Emulsion beladen z. B. durch Tropfen, Sprühen oder Drucken, anschließend getrocknet und in adäquate Größen geschnitten. Der beladene Wirkstoff ist an der Oberfläche des smartFilms adsorbiert und vor allem in den Poren eingebettet. Vorteilhaft ist, dass die Filmformung vor der Beladung abgeschlossen ist und damit beide Schritte getrennt voneinander erfolgen.

Ein weiterer Vorteil ist, dass der Einsatz von Weichmachern nicht notwendig ist. Die Beladung erfolgt auf die bereits geformten smartFilm. Die Abkopplung der Beladung vom Filmformungsschritt bietet die Möglichkeit parallel zwei oder mehrere Inhaltsstoffe insbesondere Wirkstoffe räumlich und zeitlich voneinander getrennt aufzubringen.

Vorteilhaft ist auch, dass die Unabhängigkeit des Filmformungsschrittes vom Beladungsschritt ermöglicht den oralen Film auch nach Beladung weiter zu modifizieren. So kann beispielsweise die Beladung erhöht werden durch weitere Aufbringung von Wirkstoffen und/oder Hilfsstoffen und dadurch den oralen Film seine Individualität geben (personalisierte Medizin). Im Rahmen der personalisierten Medizin ist es auch möglich in einer Apotheke aufgestellte Drucker zu nutzen, welche befüllbar mit Inhaltsstofflösung oder -dispersion in Patronen (vgl. Druckertinte) sind, die dann patientenindividuell in der Apotheke die bestimmte Dosis auf den universell einsetzbaren smartFilm drucken. Dadurch kann patientenindividuell und schnell das Arzneimittel zur Verfügung gestellt werden.

Der Vorteil beim Einsatz von smartFilms ist die Beständigkeit gegenüber Feuchtigkeit als flächiges Material. Dies erleichtert nicht nur die Herstellung, die Handhabung, die Lagerung und den Transport, sondern beeinflusst auch die Haltbarkeit des oralen Films positiv. So können aus smartFilms orale Filme als kostengünstiges und beständiges Massenprodukt hergestellt werden.

Als regenerativer, ubiquitär verfügbarer Rohstoff eignet sich Cellulose ideal für die industrielle Anwendung als papierformendes Material, insbesondere als smartFilm zur Herstellung von Filmprodukten zur oralen und peroralen Applikation. Die neutrale Gustatorik und Olfaktorik, ohne Begleitaromen eignen sich optimal für eine orale oder perorale Applikation. Dabei zeigt sich, dass die Aufnahmefähigkeit von Flüssigkeiten die positiven Eigenschaften von smartFilms zum Einsatz für orale Filme abrundet. So kann Cellulose als Faserstoff von Papier, da es inert zu den meisten Substanzen ist, als Hilfsstoff mit einer Vielzahl verschiedener Inhaltsstoffe insbesondere Wirkstoffe, ohne Inkompatibilitäten und/oder Stabilitätsminderung hoch beladen werden. Inkompatible Wirkstoffe können auch räumlich getrennt auf verschiedenen Flächenbereichen der smartFilms aufgetragen werden. Die räumliche Separierung vermeidet Inkompatibilitäten, dies ist bei der Herstellung mittels Lösungsmittelgießverfahren oder Schmelzextrusionsverfahren nicht oder nur schlecht möglich.

SmartFilms sind vielseitig für Inhaltsstoffe insbesondere Wirkstoffe in den verschiedenen wünschenswerten Charakteristika verfügbar. Hauptcharakteritika für smartFilms ist eine möglichst hohe Beladung relativ zur Gesamtmasse, eine steuerbare Freisetzung des Wirkstoffs und eine fasrige Matrixstruktur, welche die Inhaltsstoffe insbesondere Wirkstoffe, optional im amorphen Zustand beinhaltet.

Das erfindungsgemäße Herstellungsverfahren zur Beladung von smartFilms ist eine schnelle, industriell leicht adaptierbare Methode mit Erzielung einer hohen Beladung. Die etablierte Technologie der Zeitungsindustrie (Druck und Schnitt) mit einer Leistung von mehreren tausend Quadratmetern pro Minute ist eine adaptierbare Möglichkeit zur Herstellung von oralen Filmen aus smartFilms als alternative orale und perorale Applikationsform. Damit lassen sich nach Validierung des Prozesses und Qualifizierung der Maschinen Chargengrößen von mehrern Millionen oralen Filmen binnen weniger Minuten herstellen, was die erfindungsgemäße Applikationsform zu einem massenfähigen Produkt macht.

Es hat sich gezeigt, dass smartFilms mit einer möglichst hohen Flüssigkeitsaufnahmefähigkeit für eine hohe Beladung durch multiples Auftragen mit intermediäre Trocknung besonders geeignet sind. Darüber hinaus bedingt eine hohe Flüssigkeitsaufnahmefähigkeit auch eine simple Herstellung, da unproblematisch, ohne technischen Aufwand, die smartFilms mit einem beliebigen Beladungsmedium beladen werden kann.

Erfindungsgemäß hat sich gezeigt, dass Löschpapiere und Hygienepapiere (Tissue-Papiere, die für die Körperhygiene geeignet sind) sich dabei besonders eignen aufgrund ihrer Zweckbestimmung, bei welcher eine möglichst hohe Flüssigkeitsaufnahmefähigkeit gewünscht ist. Diese Flüssigkeitsaufnahmefähigkeit wird bei der erfindungsgemäßen Herstellungsmethode ausgenutzt.

So ist Löschpapier per definitionem ein Träger mit hoher Saugfähigkeit. Berücksichtigt bei der Auswahl wurde stellvertretend Löschpapier Saugstark & Sicher (dm-drogerie markt GmbH & Co. KG; Deutschland) (European Article Number (EAN): 4010355975942). Dieses Papier ist ein weißes, raues Papier bestehend aus einer einzelnen Lage.

Dem gegenüber wurden zwei Hygienepapiere hergestellt aus leichtgewichtigen, trocken oder nass gekreppten Papieren (Tissue-Papiere) mit in die Auswahl einbezogen. Tissue-Papiere zeichnen sich typischerweise durch ihre hohe Flüssigkeitsaufnahmefähigkeit aus. Stellvertretend für Tissueprodukte als smartFilms wurden einerseits die Küchentücher Saugstark & Sicher (dm-drogerie markt GmbH & Co. KG; Deutschland) (European Article Number (EAN): 4010355975942) und die Einmalwaschlappen ebelin (dm-drogerie markt GmbH & Co. KG; Deutschland) (European Article Number: 4010355654212) genutzt.

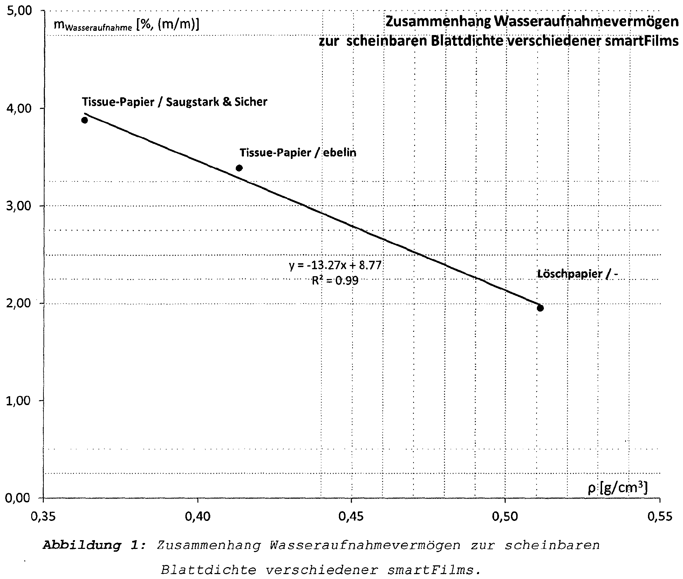

Es zeigte sich ein linearer Zusammenhang zwischen der scheinbaren Blattdichte (ρ) und dem Wasseraufnahmevermögen (m_{Wasseraufnahme}) als Surrogatparameter für die Flüssigkeitsaufnahmefähigkeit der smartFilms (siehe Abbildung 1). Dieser Zusammenhang ist ein entscheidender Faktor für die erfindungsgemäße Herstellung und die damit verbundene Auswahl der passenden smartFilms für einen bestimmten Inhaltsstoff bzw. Wirkstoff. Es zeigte sich, dass eine geringe scheinbare Blattdichte für ein möglichst hohes Flüssigkeitsaufnahmevermögen erforderlich ist.

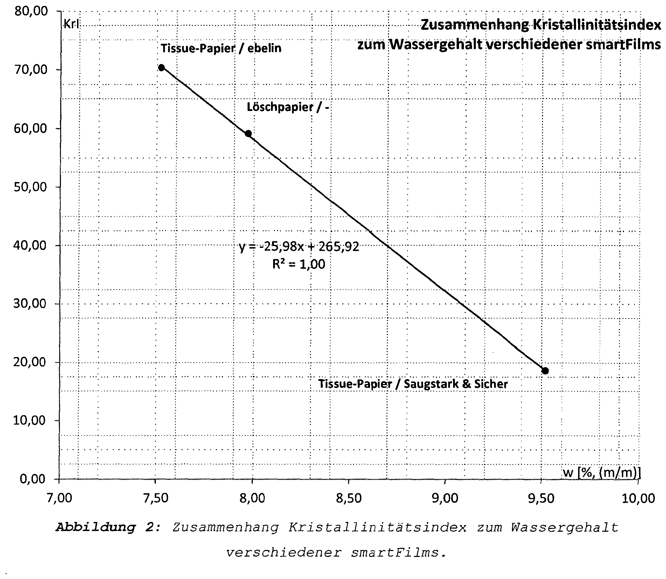

Ebenfalls zeigte sich ein linearer Zusammenhang zwischen dem Kristallinitätsindex (KrI) (nach Segal et al. (1959). Textile Research Journal, 29, 786-794.) und dem Wassergehalt (w) der smartFilms (siehe Abbildung 2). Je höher der Kristallinitätsindex, desto geringer der Wassergehalt der smartFilms. Dieser Zusammenhang kann zur Abschätzung der Stabilität feuchtigkeitslabiler Inhaltsstoffe insbesondere Wirkstoffe im oralen Filmen interessant sein.

Mit der hier beschriebenen Erfindung ist es möglich relative Beladungen deutlich über 30 % (m/m) bis zu 84,2 % (m/m) bezogen auf das Gesamtgewicht des oralen Films herzustellen. Durch die erfindungsgemäßen oralen Filme basierend auf smartFilms, sowie deren Herstellung steht ein System und Verfahren zur Verfügung, welches höhere absolute und relative Beladungen in oralen Filmen möglich macht. Dies erweitert erheblich das Spektrum der möglichen einsetzbaren Hilfsstoffe und Wirkstoffe und bietet eine industrielle verbesserte Alternative zu bisher beschriebenen oralen Filmen.

Bei einem typischen Gewicht von oralen Filmen hergestellt aus smartFilms beispielsweise von 60,0 mg pro cm² können mit den erfindungsgemäßen oralen Filmen bis zu 50,5 mg pro cm² appliziert werden. Dabei kann durch Modifizierung der applizierten Fläche des oralen Films die Dosierung gesteuert werden. So ist beispielsweise bei einer Applikation eines quadratischen oralen Films mit einer Fläche von 4 cm² eine Dosierung von bis zu 202,0 mg pro Einmaldosierung möglich.

So können orale Filme zur oralen und peroralen Applikation von Inhaltsstoffen insbesondere Wirkstoffen als Fertigprodukte beispielsweise Kosmetika, Nahrungsergänzungsmittel, diätische Lebensmittel, Medizinprodukte, Biozide und insbesondere Arzneimittel aus smartFilms durch einfache und schnelle Herstellung unter Verwendung eines geeigneten Verpackungssystems prozessiert werden.

Bevorzugt wird ein mit den gewünschten Materialeigenschaften ausgewählter smartFilm mit geeigneten flüssigen Lösungsmitteln und/oder Dispersionsmedien beladen. Flüssige Dispersionsmedien zur Herstellung der Beladungsmedien können beispielsweise Wasser, Ethanol, Propylenglykol, flüssige Polyethylenglykole, Glycerol, fette Öle sowie deren Mischungen sein ohne auf diese beschränkt zu sein. Dazu wird der ausgewählte smartFilm genommen. Der besondere Vorteil ist, dass der zeitintensive Herstellungsschritt der Filmformung entfällt, da dieser bereits abgeschlossen ist. Auch wird bei dem erfindungsgemäßen Verfahren durch die Abkopplung des Filmformungsschritts vom Beladungsschritt, eine Störung der Filmformung durch den Wirkstoff oder andere Hilfsstoffe verhindert. Besonders vorteilhaft ist, dass durch das erfindungsgemäße Herstellungsverfahren die Inhaltsstoffe insbesondere der Wirkstoff nicht thermisch durch den Filmformungsschritt belastet wird.

So werden die smartFilms über eine waagerechte Fläche gelegt und an zwei gegenüberliegenden Enden gespannt und fixiert. Dies sorgt für eine definierte plane Beladungsfläche. Anschließend werden die smartFilms mit der geeigneten Wirkstofflösung oder Wirkstoffdispersion im Winkel von 90 ° einseitig beladen getrocknet und bei Bedarf anschließend weiter beladen oder abschließend in die gewünschte Form und Größe zugeschnitten.

Beispielweise kann zur Beladung des Porenhohlraums des smartFilms mit Inhaltsstoffen insbesondere Wirkstoffen im Kleinmaßstab eine definierte Dispersionsmenge, ohne äußeren Druck, mit Hilfe einer Hubkolbenpipette durch Tropfen aufgetragen werden. Nach der anschließenden schonenden Trocknung der beladenen smartFilms bei Raumtemperatur unter Lichtausschluss erfolgt das Zuschneiden in die gewünschte finale Form als oraler Film oder ein bzw. weitere Beladungsschritt(e) bis zur gewünschten Beladung der Inhaltsstoffe insbesondere der Wirkstoffe im oralen Film. Die Trocknung bei Raumtemperatur unter gegebener Luftfeuchte ist ohne weiteren apparativen Aufbau umsetzbar.

Großtechnisch laufen smartFilms als gespannte Bahn über ein bewegliches Walzennetzwerk in welchem beladen, getrocknet und zu Rechtecken bevorzugt Quadraten, ohne Verschnitt, geschnitten wird. Bei der Massenherstellung kann auch eine automatisierte Beladung z. B. durch Sprühen gewählt werden.

Das Herstellungsverfahren bietet auch die Möglichkeit den oralen Film weiter zu modifizieren durch weitere oder parallele Auftragung von Wirk- und Hilfsstoffen. So kann beispielsweise die Freisetzung gesteuert werden z. B. durch einen magensaftresistenten Überzug bei peroral zu applizierenden Filmprodukten oder beispielsweise auch der Geschmack gesteuert werden z. B. durch einen maskierenden Überzug. Auch kann die Compliance des Patienten nicht nur durch die smartFilms, sondern auch während der Herstellung erhöht werden z. B. durch Veränderung des Aussehens durch Einfärbung.

Die erfindungsgemäße Herstellungsmethode nutzt die fertig geformten smartFilms, welche ohne Vorbehandlung und ohne weiteres Prozessieren genutzt werden kann. Es sind keine Weichmacher oder Trägerstabilisatoren zur Filmformung nötig. Die Herstellung erfolgt durch Beladung der smartFilms mit einem geeigneten Beladungsmedium. Diese Herstellungsmethode stellt einen zeit- und kosteneffizienten Prozess, welcher problemlos großtechnisch zur Massenherstellung adaptiert werden kann, dar.

Es hat sich gezeigt, dass smartFilms zur Herstellung oraler Filme und der beschriebenen Herstellungsmethode eine relative Beladung deutlich über 30 % (m/m) bezogen auf das Gesamtgewicht des oralen Films, sogar bis zu 84,2 % (m/m) möglich macht. So können, abhängig von der finalen Filmgröße, verschiedenste Wirkstoffgruppen in Form von Filmen oral oder peroral appliziert werden, wie beispielsweise nichtopiode Analgetika (z. B. Acetylsalicylsäure, Paracetamol, Ibuprofen), opioide Analgetika (z. B. Tilidin, Codein, Fentanyl), Antibiotika (z. B. Clarithromycin, Clindamycin, Minocylin), Betarezeptorenblocker (z. B. Metoprolol, Bisoprolol, Nebivolol), AT₁-Rezeptorantagonisten (z. B. Valsartan, Losartan, Candesartan), Angiotensinkonversionsenzym-Hemmer (z. B. Ramipril, Captopril, Enalapril), Diuretika (z. B. Furosemid, Hydrochlorothiazid, Spironolacton), Calciumkanalblocker (z. B. Nifedipin, Diltiazem, Verapamil), Protonenpumpenhemmer (z. B. Pantoprazol, Omeprazol, Lansoprazol), HMG-CoA-Reduktasehemmer (Simvastatin, Lovastatin, Pravastatin), Schilddrüsenhormone (z. B. L-Thyroxin), Glucocorticoide (z. B. Prednisolon, Prednison, Dexamethason), Antidepressiva (z. B. Amitryptilin, Mianserin, Fluoxetin), Neuroleptika (z. B. Clozapin, Haloperidol, Perazin), Benzodiazepine (z. B. Diazepam, Tetrazepam, Midazolam), Benzodiazepinanaloga (z. B. Zaleplon, Zopiclon, Zolpidem), Antihistaminika (z. B. Doxylamin, Cetirizin, Ranitidin, Famotidin), Lokalanästhetika (z. B. Lidocain, Tetracain, Mepivacain), Antiepileptika (z. B. Carbamazepin, Phenytoin, Valproinsäure), Antiparkinsonika (z. B. Levodopa, Benserazid, Carbidopa), Antidiabetika (z. B. Metformin, Glibenclamid, Rosiglitazon, Sitagliptin, Repaglinid) und Thrombozytenaggregationshemmer (z. B. Ticlopidin, Clopidogrel) ohne auf diese Wirkstoffgruppen und deren Beispiele beschränkt zu sein. Diese Wirkstoffe können in Form von Lösungen, Suspensionen und/oder Emulsionen auf die smartFilms aufgebracht werden. Die obligatorischen Wirkstoffe auf den smartFilms kommen neben fakultativen weiteren Inhaltstoffen zur oralen und peroralen Applikation für Kosmetika, Nahrungsergänzungsmittel, diätische Lebensmittel, Medizinprodukte, Biozide und insbesondere Arzneimittel zum Einsatz ohne auf diese beschränkt zu sein.

Die Beladung erfolgt in mehreren Zyklen mit intermediärer Trocknung auf verschiedenen smartFilms. In Tabelle 1 findet sich die Übersicht ausgewählter hergestellter Filme.

**Tabelle 1: Übersicht der Beispiele zur Herstellung von Filmprodukten zur oralen und peroalen Applikation**

| **Beispiel** | **Wirkstoff** | **Beladungsmedium** | **Massenanteil des Wirkstoffs im Beladungsmedium [%, m/m]** | **Volumen pro Beladung [µl]** | **Anzahl an Beladungen** | **relative Beladung des oralen Films bezogen auf das Gesamtgewicht des oralen Films [%, m/m]** |
|---|---|---|---|---|---|---|
| ***smartFilm: Einmalwaschlappen*** / ***ebelin*** | | | | | | |
| 1 | Folsäure | Lösung (wässrige Natriumcarbonatlösung) | 2,5 | 90 | 50 | 47,2 |
| 2 | Diphenhydraminhydrochlorid | Lösung (Wasser) | 40,0 | 44 | 4 | 82,0 |
| 7 | Coenzym-Q10 | Lösung (Diethylether) | 5,0 | 45 | 10 | 12,2 |
| 8 | Coenzym-Q10 | Lösung (Cetylpalmitat) | 10,0 | so viel wie nötig | 1 | 5,4 |
| 9 | Coenzym-Q10 | Lösung (Cetylpalmitat) | 40,0 | so viel wie nötig | 1 | 24,4 |
| 10 | Rutin | Mikrosuspension | 5,0 | 45 | 10 | 13,4 |
| 11 | Rutin | Nanosuspension | 5,0 | 45 | 50 | 36,6 |
| 12 | Coenzym-Q10 | Nanoemulsion | 2,5 | 90 | 10 | 10,4 |
| 13 | Hesperidin | Nanosuspension | 5,0 | 45 | 10 | 13,0 |
| 14 | Coenzym-Q10 | Nanosuspension | 2,0 | 112,5 | 10 | 12,6 |
| 15 | Folsäure* | Polymer-dispersion | 0,25 | so viel wie nötig | 1 | 0,3 |

| *eingebettet mit Ammoniummethacrylat-Copolymer (Typ A) | | | | | | |
|---|---|---|---|---|---|---|
| 16 | Retinol | Nanolipidsuspension | 4,8 | 46,9 | 10 | 7,54 |
| 17 | Coenzym-Q10 | Nanolipidsuspension (1) | 4, 8 | 46,9 | 10 | 8, 96 |
| 18 | Coenzym-Q10 | Nanolipidsuspension (2) | 4, 8 | 46,9 | 10 | 6, 53 |
| 19 | Coenzym-Q10 | Nanolipidsuspension (3) | 4,8 | 46,9 | 10 | 8,38 |

| ***smartFilm:* Küchentücher / *Saugstark & Sicher*** | | | | | | |
|---|---|---|---|---|---|---|
| 3 | Folsäure | Lösung (wässrige Natriumcarbonatlösung) | 2,5 | 90 | 10 | 35,6 |
| 4 | Diphenhydraminhydrochlorid | Lösung (Wasser) | 40,0 | 19 | 4 | 84,2 |

| ***smartFilm: Löschpapier* / -** | | | | | | |
|---|---|---|---|---|---|---|
| 5 | Folsäure | Lösung (wässrige Natriumcarbonatlösung) | 2,5 | 90 | 10 | 18,8 |
| 6 | Diphenhydraminhydrochlorid | Lösung (Wasser) | 40,0 | 18 | 4 | 75, 4 |

Die aufgetragene Menge pro Beladung der jeweiligen stabilen Wirkstoffdispersion wurde in den Beispielen 1, 3, 5, . 7, 10-14 und 16-19 auf einen Massenanteil von 2,5 % (m/m) in 90 µl Beladungsmedium normiert. Diese definierte Wirkstoffbeladung sichert die Vergleichbarkeit der einzelnen Beladungen mit den verschiedenen Inhaltsstoffen insbesondere Wirkstoffen untereinander. So konnte mit dieser Herstellungsmethode bereits eine Beladung mit einem Wirkstoffgehalt von 47,2 % (m/m) mit Folsäurelösung (siehe Beispiel 1) und 36,6 % (m/m) mit Rutinnanosuspension (siehe Beispiel 11) erzielt werden.

Zur zusätzlichen Steuerung der Wirkstofffreisetzung wurde Folsäure in eine Dispersion aus Ammoniummethacrylat-Copolymer (Typ A) eingearbeitet woraus sich nach Trocknung eine Matrix bildete, in welcher der Wirkstoff Folsäure eingebettet vorlag. So zeigte sich, dass sowohl Suspensionen, Emulsionen als auch Lösungen als Beladungsmedium verwendet werden können.

Darüber hinaus wurden die drei beschriebenen smartFilms viermal mit einer vierzigprozentigen wässrigen Diphenhydraminhydrochloridlösung mit unterschiedlichen Beladungsvolumina beladen. Dabei wurde eine finale relative Beladung von 75,4 % (m/m) für das Löschpapier (siehe Beispiel 6), 84,2 % (m/m) für die Küchentücher (siehe Beispiel 4) und 82,0 % (m/m) für die Einmalwaschlappen (siehe Beispiel 2) erzielt. Dies entspricht einer Erhöhung der Beladungsmasse relativ zur Ausgangsmasse des jeweiligen smartFilms von 305 % (m/m) für Beispiel 6, 532 % (m/m) für Beispiel 4 und 455 % (m/m) für Beispiel 2.

Ebenso verhält es sich, wenn die Beladung mit einer verflüssigten Coenzym-Q10-Mischung erfolgt, die sich unmittelbar nach dem Auftragen auf dem smartFilm verfestigt, wie aus Tabelle 1 ersichtlich. Dazu wurde die homogene Mischung aus Cetylpalmitat und Coenzym Q10 vollständig auf 55 °C (Schmelzbereich Cetylpalmitat: T = 45 bis 52 °C) erwärmt und die entstandene Lösung noch fließfähigen Zustand homogen auf dem smartFilm verteilt. Die Auftragung verschiedener Wirkstoffe mit Hilfstoffen als Lipidsuspension und Coenzym-Q10 als Lösung in einem Lipid erfolgt zum Schutz des beladenen Wirkstoffs. Der nicht eingebettete Wirkstoff, welcher sich adsorbiert an der Oberfläche des smartFilms befindet wird durch das entsprechend umgebende Lipid vor äußeren Einflüssen geschützt.

Durch Erhöhung des Massenanteils an Wirkstoff im Beladungsmedium, durch Vergrößerung der Fläche des smartFilms und/oder Erhöhung der Menge des Beladungsmediums kann die Beladungsanzahl gesenkt und/oder die absolute Beladungsmenge und die relative Beladung bezogen auf das Gesamtgewicht erhöht werden. Dies eröffnet die Nutzung verschiedener Wirkstoffe und deren Beladungsdispersionen für die Applikationsform oraler Film, welche nicht mehr auf bestimmte ausgewählte hochpotente Wirkstoffe limitiert ist.

Es hat sich gezeigt, dass sich die weitgehend inerten smartFilms als Aufsaugmedium zur Beladung eignen. Die Beladung mit Inhaltsstoffen insbesondere Wirkstoffe erfolgt dabei eingebettet in das Porenvolumen und nach Sättigung des Porenvolumens in der Matrix auch adsorbiert an der Oberfläche der smartFilms.

Die beschriebene Herstellungsmethode ist geeignet aus smartFilms orale Filme herzustellen und dabei die smartFilms mit verschiedenen Inhaltsstoffen insbesondere Wirkstoffen in verschiedenen Beladungsdispersionen zu beladen. Es können erfindungsgemäß orale Filme mit bisher nicht erreichter relativer Beladung von bis zu 84,2 % (m/m) bezogen auf das Gesamtgewicht des oralen Films hergestellt werden, so dass das Spektrum an geeigneten Wirkstoffen entscheidend erweitert wurde. Der besondere Vorteil ist, dass neben dem smartFilm und der Beladungsdispersion keine weiteren Hilfsstoffe notwendig sind. Nichtsdestotrotz können, ohne Probleme, weitere Inhaltsstoffe beispielsweise zur Erhöhung der Compliance oder Steuerung der Freisetzung zum Einsatz kommen.

Die Wirkstofffreisetzung oraler Filme hergestellt aus smartFilms ist mit der Freisetzung aus klassischen oralen Applikationsformen z. B. Tabletten, aber auch bisherigen oralen Filmen vergleichbar oder dieser überlegen. So ist es möglich die Freisetzung zu kontrollieren und diese zu steuern. So sind für orale Filme basierend auf smartFilms Freisetzungsprofile abgestimmt auf den Wirkstoff und dessen Pharmakodynamik und Pharmakokinetik, als anwenderorientierte Applikationsform, möglich. Möglichkeiten bestehen beispielsweise in der Verlängerung, der Beschleunigung, der Verzögerung und/oder Steuerung des Freisetzungsprofils nach einer bestimmten Kinetik. Die Freisetzung kann erfindungsgemäß angepasst werden an den Wirkstoff und dessen Pharmakodynamik und -kinetik. Dieses wird durch die Wahl des Materials der smartFilms und/oder weitere Bearbeitung beispielsweise durch Ummantelung gesteuert. Kontrolle der Freisetzung ist ebenfalls möglich durch Beladung der Filme mit Matrixpartikeln wie Polymermikro- und nanopartikel, Lipidmikro- und nanopartikel, anorganische poröse Partikel wie Silica z. B. Syloid^{®} (W. R. Grace and Company; USA) oder Neusilin^{®} (Fuji Chemical Industries Co., Ltd.; Japan). Die Freisetzungsgeschwindigkeit wird dann von den Eigenschaften der Partikelmatrix bestimmt (z. B. Viskosität, Porosität, Oberfläche, Durchmesser des Partikels als relevante Diffusionsstrecke für die Moleküle).

Vorteilhaft dabei ist, dass die Freisetzung der Inhaltsstoffe insbesondere der Wirkstoffe aus dem oralen Film im Vergleich zu herkömmlichen oralen Applikationsformen aufgrund der vergrößerten Oberfläche relativ zur Gesamtmasse schneller erfolgt. Darüber hinaus beginnt die Freisetzung des oralen Films sofort bei Kontakt mit den Verdauungsflüssigkeiten durch Auswaschung aus dem smartFilm, wohingegen beispielsweise eine Tablette erst zerfallen muss oder derzeitige orale Filme erst schmelzen oder sich auflösen müssen um anschließend den Wirkstoff freizusetzen. Die Freisetzung erfolgt nach Ingestion ohne Zeitverzögerung durch den Zerfall der Applikationsform. Ein Abbau während der Verdauung erfolgt nicht oder nur begrenzt. Der smartFilm bleibt während des Passierens des gesamten Verdauungstraktes zumindest überwiegend erhalten, wobei ggf. eine mechanische Zerkleinerung bzw. ein Zerfasern aufgrund der Peristaltik möglich ist. Der Geschwindigkeitsvorteil bedingt durch die sofortige Auswaschung der Inhaltsstoffe ist besonders bei Wirkstoffen von Vorteil, bei welchen es zu einer schnellen Wirkung kommen soll, z. B. Analgetika oder Antihistaminika. Es kommt zu einer stoßartigen sofortigen Freisetzung (engl. burst release) der Inhaltsstoffe, welche schnell und quantitativ zur Resorption zur Verfügung stehen.

Überraschend wurde gefunden, dass nach Evaporation des Lösungsmittels bzw. Dispersionsmediums bei nicht zu hoher Konzentration des Wirkstoffes dieser im amorphen Zustand vorlag. Dies ist entgegen dem bisherigen Stand der Technik. Es ist bekannt, dass Inhaltsstoffe insbesondere Wirkstoffe im amorphen Zustand erhalten werden können, wenn die Lösung oder das Dispersionsmedium in Mesoporen aufgesaugt und anschließend das Lösungsmittel oder das Dispersionsmedium abgedampft wird. Der Inhaltsstoff insbesondere der Wirkstoff präzipitiert, und weil aufgrund der engen Poren der Platz zur Bildung eines Kristalls fehlt, fällt er amorph aus. Die Mesoporen haben allerdings einen Durchmesser von lediglich 2-50 nm. Die Poren im smartFilm sind jedoch teilweise wesentlich größer als Makroporen bis mindestens 235 µm (siehe Beispiel 23), so dass eine Erzeugung eines amorphen Zustandes nicht zu erwarten war.

Zumindest in den größeren Poren wäre Kristallbildung zu erwarten gewesen. Diese Kristalle hätten dann als Impfkristalle fungiert und eine fortschreitende Kristallisation bewirkt. Überraschend war dies nicht der Fall. Zur Kristallbildung kommt es nur dann, wenn die Poren gefüllt sind und sich die Inhaltsstoffe insbesondere Wirkstoffe primär auf der Oberfläche des Films anlagern, und eine Auskristallisation in einem dicken adsorbierten Inhaltsstofffilm auf der Oberfläche der smartFilms erfolgt.

Zur Erzeugung und Aufrechterhaltung des amorphen Zustandes werden in der Größe einheitliche Poren als günstig betrachtet, z. B. Silica (Formac Pharmaceuticals NV; Belgien) mit einheitlichen Porengrößen (monodispers). Im Gegensatz dazu sind die Poren in ihrer Größe in den erfindungsgemäßen smartFilms sehr polydispers, was die Kristallbildung entsprechend der Lehrmeinung fördern sollte.

Poröse Materialien zur Erzeugung eines amorphen Zustandes in ihren Poren werden normalerweise optimiert bezüglich relevanter Eigenschaften hergestellt, z. B. Porengröße, Einheitlichkeit der Porendurchmesser, Porenvolumen, funktionelle Gruppen an der Oberfläche (z. B. Formac^{®}-Silikate, Syloid^{®}, Neusilin^{®} etc.). Im Gegensatz dazu wurde dies an den erfindungsgemäßen smartFilms nicht vorgenommen. Optimierung erfolgte in Bezug anderer Eigenschaften wie z. B. der Reißfestigkeit bei Hygienepapieren, Saugfähigkeit bei Löschpapier mit größeren Poren und kostengünstige Herstellung. Vorhandene Eigenschaften wie relativ große Poren, Polydispersität der Poren sprachen gegen eine mögliche Erzeugung eines amorphen Zustandes. Überraschenderweise wurde dieser amorphe Zustand aber erhalten, und war langzeitstabil über 12 Monate (siehe Beispiel 22). Ein amorpher Zustand im Sinne der vorliegenden Erfindung liegt vor wenn im Röntgendiffraktogramm ein amorpher Halo ohne kristalline Peaks erhalten wird.

Die erfindungsgemäßen smartFilms wurde auch mit Nanokristallen in Form von Nanosuspensionen beladen (Suspensionen von Nanokristallen in z. B. wässriger Tensidlösung). Nanokristalle im Sinne dieser Erfindung sind Partikel aus reinem Wirkstoff oder Wirkstoffgemischen ohne Anwesenheit eines Matrixmaterials (z. B. Polymere oder Lipide) mit einer mittleren Partikelgröße im Bereich zwischen 1 nm bis < 1.000 nm. Die Partikelgröße wurde berechnet aufgrund Bestimmung der Anzahlverteilung mittels Photonenkorrelationsspektroskopie (Messung der Intensitätsverteilung und anschließende Konvertierung in die Anzahlverteilung).

Die smartFilms können auch mit MikrometerKristallen, in der Regel im unteren Mikrometerbereich (< 500 µm), bevorzugt kleiner als 200 µm (= ca. häufige Größe der maximalen Poren), insbesondere aber mit Kristallen im Bereich < 50 µm bis 1 um beladen werden. Über die Kristallgröße und die damit korrelierende Oberfläche kann die Auflösungsgeschwindigkeit kontrolliert werden. Mikrometerkristalle werden dann verwendet, wenn eine langsamere, verzögerte Auflösungsgeschwindigkeit im Vergleich zu Nanokristallen eingestellt werden soll. Es ist auch die Kombination von Nanokristallen mit Mikrometerkristallen möglich, z. B. initiale starke Freisetzung (engl. burst release) durch Nanokristalle gefolgt von einer prolongierten Freisetzung aus Mikrometerkristallen.

Die erfindungsgemäßen smartFilms können auch mit anderen Nanopartikeln als Nanokristallen beladen werden. Allgemeine Definition von Nanopartikel im Sinne dieser Erfindung: Nanopartikel sind Partikel aus Wirkstoff oder Wirkstoffgemischen, optional in Kombination mit einem weiteren oder mehreren Hilfsstoffen (z. B. Polymere und Lipide als Matrixmaterial, Tenside zur Lösungsvermittlung von Wirkstoffen) mit einer mittleren Partikelgröße im Bereich zwischen 1 nm bis < 1.000 nm, Partikelgröße berechnet aufgrund Bestimmung der Anzahlverteilung mit Photonenkorrelationsspektroskopie (Messung der Intensitätsverteilung und anschließende Konvertierung in die Anzahlverteilung). Derartige Nanopartikel sind z. B. aber nicht ausschließlich Polymernanopartikel, Liposomen, Cubosomen, Nanoemulson, Lipidnanopartikel (solid lipid nanoparticles - SLN, nanostructured lipid carriers - NLC, smartLipids) und Mizellen aus Tensiden und Polymeren. Beispielhaft wurden die smartFilms mit Lipidnanopartikeln beladen, identisch dazu kann die Beladung mit anderen Nanopartikel erfolgen. Beladung mit Mizellen hat den Vorteil, dass aufgrund der Lösungsvermittlung sehr schnelle Freisetzung aus den smartFilms erzielt werden kann. Die smartFilms können auch mit den Mizellen ähnlichen Systemen wie Mikroemulsionen (Wasser, Öl, Tensid oder Tensidmischung) oder selbstemulgierenden Systemen (Öle, Tenside, Wasser) beladen werden. Anstatt Beladung mit Mikroemulsion oder selbstemulgierendem System kann die Beladung auch mit deren Präkonzentraten erfolgen, die erst bei Kontakt mit Wasser (z. B. in der Mundhöhle) zur Bildung einer Mikroemulsion oder eines selbstemulgierenden Systems führen.

Nanopartikel, die ein Matrixmaterial (z. B. Polymer, Lipid) enthalten werden dann bevorzugt verwendet, wenn chemisch labile Wirkstoffe gegen Zersetzung geschützt werden sollen. Die Partikelmatrix vermindert schädliche äußere Einflüsse von z. B. Sauerstoff und Licht. Mit der Matrix kann optional auch eine Freisetzung verzögert werden, da die Wirkstoffe zunächst durch das feste Matrixmaterial diffundieren müssen (Feststoffdiffusion), um freigesetzt (gelöst) zu werden. Des Weiteren kann durch das aufgebrachte Matrixmaterial der Porendurchmesser monodispers gehalten werden.

Neben der Verwendung als finale Arzneiform für Arzneimittel oder finale Applikationsform für andere Produkte (z. B. Nutraceuticals oder Medizinprodukte), z.B. als oraler Film, können die erfindungsgemäßen smartFilms auch als Zwischenprodukt für die Herstellung unterschiedlicher Applikationsformen verwendet werden. Ziel ist dabei die Ausnutzung der besonderen Charakteristika der smartFilms z. B. der Freisetzungseigenschaften und/oder der Konservierung des amorphen Zustands in Kombination mit anderen Applikationsformen.

Solche Applikationsformen sind beispielsweise, ohne auf diese beschränkt zu sein, Tabletten, Kapseln, oder in der Mundhöhle zerfallende Filme (sog. orodispersible films), Kaugummi, Gele (dermal, oral oder peroral), Cremes. Generell können Sie mit praktisch allen festen, halbfesten oder flüssigen Applikationsformen kombiniert werden.

Zur Einarbeitung in diese Applikationsformen müssen die Trägermatrices typischerweise in kleinerer Form vorliegen als bei Verwendung als eigentliche Applikationsform, z. B. anstelle eines 1 cm x 1cm großen oralen Films müssen die smartFilms in kleineren Stücken vorliegen, z. B. 1 mm x 1 mm oder kleiner. Dies kann dadurch erzeugt werden, dass die smartFilms vor der Beladung in kleiner Form vorliegen, oder die oralen Filme nach dem Beladen fein zerschnitten oder gehäckselt werden (schreddern des smartFilms), sogenanntes smartFilm-Pulver.

Die Freisetzungseigenschaften bleiben im Wesentlichen erhalten, da die entstehenden Schnittkantenflächen nur einen kleinen Beitrag zur Gesamtoberfläche leisten, die letztendlich einer der freisetzungsbestimmenden Parameter ist. Sehr wesentlich ist, dass der kristalline Status der Inhaltsstoffe der oralen Filme erhalten bleibt, z. B. der amorpher Zustand von Wirkstoffen. Das smartFilm-Pulver ist somit eine ideale Form Inhaltsstoffe insbesondere Wirkstoffe stabil in andere Arzneiformen einzuarbeiten. Analoges gilt für Nanokristalle. In einer üblichen pulverförmigen Tablettenmischung kann es unter Pressdruck zum Fusionieren der Kristalle kommen, auf den erfindungsgemäßen smartFilms sind sie fest fixiert, und durch die Verteilung eingebettet in die Matrix und adsorbiert auf der Oberfläche räumlich getrennt.

Dem smartFilm-Pulver können Mischungen zur Tablettierung (Pulvern, Granulaten) zugemischt werden, oder auch direkt verpresst werden (siehe Beispiel 25), ggf. unter Zumischung von Fließregulierungsmitteln wie beispielsweise hochdisperser Kieselsäure. Cellulose Filme sind in der Lage fest Komprimate zu formen.

SmartFilm-Pulver kann in Kapseln abgefüllt werden, entweder als trockenes Pulver oder als nichtwässrige Suspension. Sie können in Gelen oder Cremes durch intensives Rühren dispergiert werden. Als feines Pulver können Sie auch in andere traditionelle Filme wie Wafer eingearbeitet werden. Kaugummis können zur Freisetzung von Wirkstoffen in der Mundhöhle verwendet werden, klassisches Beispiel ist Nikotin. Das SmartFilm-Pulver kann der Kaugummimischung vor dem Verpressen oder Extrudieren einfach zugemsicht werden. Generell können die SmartFilm-Pulver in alle Applikationsformen eingearbeitet werden, in die pulverförmige Substanzen eingearbeitet werden können.

Die erfindungsgemäßen smartFilms eignen sich auch zur Beladung mittels 3D-Druck (sog. 3D-printing). Hier können nach Füllung des Porenvolumens mehrere Schichten auf den Film aufgebaut werden. Die Struktur der smartFilms stabilisiert diese dreidimensionalen Gebilde.

### Beispiele

Die Erfindung soll nachstehend an mehreren Ausführungsbeispielen näher erläutert werden.

### Beschreibung der bevorzugten Ausführungsformen

Die in den folgenden Beispielen 1, 2, 3, 4, 6, 11 beschriebenen Ausführungen stellen erfindungsgemäß den präferierten smartFilm und die funktionierend erprobten Herstellungsverfahren stabiler, hoch und homogen beladender oraler Filme dar, ohne aber auf diese beschränkt zu sein.

### Beispiel 1

Orale Filme hoch beladen mit Folsäure wurden hergestellt, in dem ein sechslagiges Tissue-Papier Einmalwaschlappen ebelin (dm-drogerie markt GmbH & Co. KG; Deutschland) (European Article Number: 4010355654212) mit einer scheinbaren Blattdichte von ρ = 0,413 g/cm³, einem durchschnittlichen Porendurchmesser von d_{Pore} = 21,3 µm, einem Trocknungsverlust (105 °C; bis zur Massenkonstanz: |mₙ-mₙ₊₁| ≤ 0,5 mg) von w = 7,52 % (m/m), einem Wasseraufnahmevermögen von m_{Wasseraufnahme} = 339 % (m/m) und einem Kristallinitätsindex von KrI = 70,33 als smartFilm genutzt wurde.

Ein definiertes Stück des smartFilms als Absorptionsgrundlage wurde, durch zwei an gegenüberliegenden Seiten angebrachten 50g-Gewichten, über eine waagerechte Fläche leicht gespannt und fixiert. Die Beladung erfolgte einseitig im Winkel von 90 ° unmittelbar über der Beladungsfläche mit einer Hubkolbenpipette (Biotech, Deutschland)) homogen ohne äußeren Druck, durch mengendefiniertes Tropfen.

Als Beladungsflüssigkeit wurde eine klare Folsäurelösung (in wässriger Natriumcarbonatlösung) verwendet. Dafür wurde Natriumcarbonatdecahydrat (Caesar & Loretz GmbH, Deutschland) in demineralisierten Wasser durch Mischen vollständig gelöst (28.6 g/l). Dann wurde Folsäure (Th. Geyer GmbH & Co. KG, Deutschland) als Reinstoff in dieser vorbereiteten Natriumcarbonatlösung, durch Mischen, klar gelöst zu einer finalen Konzentration von 2,5 % (m/m). Folsäure liegt deprotoniert vor und färbt die Lösung zu einer orangen bis leicht rötlichen Flüssigkeit.

90 µl dieser wässrigen Folatlösung wurden direkt auf die Beladungsfläche pro Beladung homogen verteilt aufgetragen mit einer Hubkolbenpipette (10-100 µl). Zwischen den einzelnen Beladungen und nach finaler Beladung wurde der beladene smartFilm bei Raumtemperatur schonend unter Lichtausschluss getrocknet. Der gesamte Herstellungsprozess erfolgte unter Raumklima (T = 25.9 ± 3.0 °C; rF = 44 ± 13 %). Verschiedene homogen beladene orale Filme mit fünf- bis fünfzigfacher Beladung (Fünferschritte) mit intermediärer Trocknung wurden hergestellt. Der durchschnittliche Massenzuwachs des Wirkstoffs pro Beladung lag bei 0,25 mg/cm² und die relative Beladung bezogen auf das Gesamtgewicht des fünfzigfach beladenen oralen Films bei 47,2 % (m/m). Abschließend wurden die homogen beladenen oralen Filme in ihre finale Größe von 1 cm² (1 × 1 cm), fertig zur Verpackung und Anwendung, zu geschnitten. Die oralen Filme wurden unter Minimierung von Licht, Luftfeuchte und Luftsauerstoff bei Temperaturen zwischen 20 und 30 °C gelagert.

### Beispiel 2

Orale Filme hoch beladen mit Diphenhydraminhydrochlorid wurden analog zu Beispiel 1 hergestellt.

Hingegen wurde im Vergleich zu Beispiel 1 eine klare wässrige Diphenhydraminhydrochloridlösung als Beladungsmedium genutzt. Dafür wurde Diphenhydraminhydrochlorid (Beijing Taiyang Pharmaceutical Industry Co., Ltd., China) in demineralisierten Wasser durch Mischen vollständig klar gelöst zu einer finalen Konzentration von 40,0 % (m/m).

44 µl dieser wässrigen Diphenhydraminhydrochloridlösung wurden direkt auf die Beladungsfläche pro Beladung homogen verteilt aufgetragen. Der gesamte Herstellungsprozess erfolgte unter Raumklima (T = 26,2 ± 0,8 °C; rF = 44 ± 5 %). Homogen beladene orale Filme mit vierfacher Beladung mit intermediärer Trocknung wurden hergestellt.

Der durchschnittliche Massenzuwachs des Wirkstoffs pro Beladung lag bei 17,06 mg/cm² und die relative Beladung bezogen auf das Gesamtgewicht des vierfach beladenen oralen Films bei 82,0 % (m/m).

### Beispiel 3

Orale Filme hoch beladen mit Folsäure wurden analog zu Beispiel 1 hergestellt.

Hingegen wurde im Vergleich zu Beispiel 1 ein anderer smartFilm als Absorptionsgrundlage verwendet. Ein dreilagiges Tissue-Papier Saugstark & Sicher (dm-drogerie markt GmbH & Co. KG; Deutschland) (European Article Number (EAN): 4010355975942) mit einer scheinbaren Blattdichte von ρ = 0,363 gcm³, einem durchschnittlichen Porendurchmesser von d_{Pore} = 50,0 um, einem Trocknungsverlust von w = 9,52 % (m/m), einem Wasseraufnahmevermögen von m_{Wasseraufnahme} = 388 % (m/m) und einem Kristallinitätsindex von KrI = 18,53 wurde als smartFilm zur Herstellung oraler Filme genutzt.

Der gesamte Herstellungsprozess erfolgte unter Raumklima (T = 28.5 ± 0.9 °C; rF = 42 ± 6 %). Homogen beladene orale Filme mit fünf- und zehnfacher Beladung (Fünferschritte) mit intermediärer Trocknung wurden hergestellt.

Der durchschnittliche Massenzuwachs des Wirkstoffs pro Beladung lag bei 0,28 mg/cm² und die relative Beladungskonzentration des zehnfach beladenen oralen Films bei 35,6 % (m/m).

### Beispiel 4

Orale Filme hoch beladen mit Diphenhydraminhydrochlorid wurden analog zu Beispiel 3 hergestellt.

Hingegen wurde im Vergleich zu Beispiel 3 eine klare wässrige Diphenhydraminhydrochloridlösung als Beladungsmedium genutzt. Dafür wurde Diphenhydraminhydrochlorid (Beijing Taiyang Pharmaceutical Industry Co., Ltd., China) in demineralisierten Wasser durch Mischen vollständig klar gelöst zu einer finalen Konzentration von 40,0 % (m/m).

19 µl dieser wässrigen Diphenhydraminhydrochloridlösung wurden direkt auf die Beladungsfläche pro Beladung homogen verteilt aufgetragen. Der gesamte Herstellungsprozess erfolgte unter Raumklima (T = 26,2 ± 0,8 °C; rF = 44 ± 5 %). Homogen beladene orale Filme mit vierfacher Beladung mit intermediärer Trocknung wurden hergestellt.

Der durchschnittliche Massenzuwachs des Wirkstoffs pro Beladung lag bei 7,48 mg/cm² und die relative Beladung bezogen auf das Gesamtgewicht des vierfach beladenen oralen Films bei 84,2 % (m/m).

### Beispiel 5

Orale Filme beladen mit Folsäure wurden analog zu Beispiel 3 hergestellt.

Hingegen wurde im Vergleich zu Beispiel 3 ein anderer smartFilm als Absorptionsgrundlage verwendet. Ein einlagiges Löschpapier Saugstark & Sicher (dm-drogerie markt GmbH & Co. KG; Deutschland) (European Article Number (EAN): 4010355975942 mit einer scheinbaren Blattdichte von ρ = 0,511 g/cm³, einem durchschnittlichen Porendurchmesser von d_{Pore} = 57,1 um, einem Trocknungsverlust von w = 7,97 % (m/m), einem Wasseraufnahmevermögen von m_{Wasseraufnahme} = 195 % (m/m) und einem Kristallinitätsindex von KrI = 59,15 wurde als smartFilm zur Herstellung oraler Filme genutzt.

Der durchschnittliche Massenzuwachs des Wirkstoffs pro Beladung lag bei 0,24 mg/cm² und die relative Beladung bezogen auf das Gesamtgewicht des zehnfach beladenen oralen Films bei 18,8 % (m/m).

### Beispiel 6

Orale Filme hoch beladen mit Diphenhydraminhydrochlorid wurden analog zu Beispiel 5 hergestellt.

Hingegen wurde im Vergleich zu Beispiel 5 eine klare wässrige Diphenhydraminhydrochloridlösung als Beladungsmedium genutzt. Dafür wurde Diphenhydraminhydrochlorid (Beijing Taiyang Pharmaceutical Industry Co., Ltd., China) in demineralisierten Wasser durch Mischen vollständig klar gelöst zu einer finalen Konzentration von 40,0 % (m/m).

18 µl dieser wässrigen Diphenhydraminhydrochloridlösung wurden direkt auf die Beladungsfläche pro Beladung homogen verteilt aufgetragen. Der gesamte Herstellungsprozess erfolgte unter Raumklima (T = 26,2 ± 0,8 °C; rF = 44 ± 5 %). Homogen beladene orale Filme mit vierfacher Beladung mit intermediärer Trocknung wurden hergestellt.

Der durchschnittliche Massenzuwachs des Wirkstoffs pro Beladung lag bei 7,13 mg/cm² und die relative Beladung bezogen auf das Gesamtgewicht des vierfach beladenen oralen Films bei 75,4 % (m/m).

### Beispiel 7

Orale Filme beladen mit Coenzym-Q10 wurden analog zu Beispiel 1 hergestellt.

Hingegen wurde im Vergleich zu Beispiel 1 eine klare Coenzym-Q10-Lösung verwendet. Coenzym-Q10 (Dr. Rimpler GmbH, Deutschland) als Reinstoff wurde in Diethylether (Sigma-Aldrich Co. LLC., USA) klar gelöst zu einer finalen Konzentration von 5,0 % (m/m). Coenzym-Q10 färbt die Lösung zu einer dunkelorange bis rötlichen Flüssigkeit.

45 µl der nicht-wässrigen Coenzym-Q10-Lösung wurden direkt auf die Beladungsfläche pro Beladung homogen verteilt aufgetragen. Der gesamte Herstellungsprozess erfolgte unter Raumklima (T = 23.4 ± 1.3 °C; rF = 40 ± 2 %). Homogen beladene orale Filme mit fünf- und zehnfacher Beladung (Fünferschritte) mit intermediärer Trocknung wurden hergestellt. Der durchschnittliche Massenzuwachs des Wirkstoffs pro Beladung lag bei 0,19 mg/cm² und die relative Beladung bezogen auf das Gesamtgewicht des zehnfach beladenen oralen Films bei 12,2 % (m/m).

### Beispiel 8

Orale Filme beladen mit Coenzym-Q10 wurden analog zu Beispiel 7 hergestellt.

Hingegen wurde im Vergleich zu Beispiel 7 Coenzym-Q10 (Dr. Rimpler GmbH, Deutschland) als Reinstoff in geschmolzenem Cetylpalmitat (Caesar & Loretz GmbH, Deutschland), durch Mischen bei 55 °C klar gelöst zu einer finalen Konzentration von 10,0 % (m/m). Coenzym-Q10 färbt die Lösung zu einer rötlichen Flüssigkeit. Die erwärmte Lösung wird im noch fließfähigen Zustand, vor dem Erstarren mit einem Rakel unter konstantem Druck über die Beladungsfläche verteilt.

Nach dem Erstarren des Cetylpalmitats ergab sich eine feste Lösung als Beladung auf dem smartFilm. Der smartFilm besaß nach Beladung einen durchschnittlichen Schichtdickenzuwachs von 164 µm, bei einer anfänglichen Dicke von 346 µm.

Nach einmaliger Beladung wurde der beladene smartFilm bei Raumtemperatur schonend unter Lichtausschluss erstarren gelassen. Der finale orale Film war starr und fest mit wachsiger Oberfläche auf der Beladungsseite, auf welcher der Wirkstoff Coenzym-Q10 homogen eingebettet war. Der gesamte Herstellungsprozess erfolgte unter Raumklima (T = 22.9 °C; rF = 44 %).

Der Massenzuwachs des Wirkstoffs pro Beladung lag bei 1,65 mg/cm² und die relative Beladung bezogen auf das Gesamtgewicht des beladenen oralen Films bei 5,4 % (m/m).

### Beispiel 9

Orale Filme beladen mit Coenzym-Q10 wurden analog zu Beispiel 8 hergestellt.

Hingegen wurde im Vergleich zu Beispiel 8 eine klare Lösung mit erhöhter Konzentration von Coenzym-Q10 (40 %, m/m) und erniedrigter Konzentration von Cetylpalmitat (60 %, m/m) als Beladungsflüssigkeit genutzt.

Der smartFilms besaß nach Beladung einen durchschnittlichen Schichtdickenzuwachs von 204 um, bei einer anfänglichen Dicke von 346 µm.

Der Massenzuwachs des Wirkstoffs pro Beladung lag bei 8,91 mg/cm² und die relative Beladung bezogen auf das Gesamtgewicht des beladenen oralen Films bei 24,4 % (m/m).

### Beispiel 10

Orale Filme beladen mit Rutin wurden analog zu Beispiel 1 hergestellt.

Hingegen wurde im Vergleich zu Beispiel 1 eine stabile Mikrosuspension von Rutin als Beladungsflüssigkeit verwendet. Zu diesem Zweck wurde der Wirkstoff Rutin (Denk Ingredients, Deutschland), in einer Konzentration von 5,0 % (m/m) mit 2,0 % (m/m) Polysorbat 80 (VWR International GmbH, Deutschland) als stabilisierendes Tensid, 1,0 % (m/m) gelöstem Phenoxyethanol in Ethylhexylglycerol (9:1) (Euxyl PE9010, Schülke & Mayr GmbH, Deutschland) als Konservierungsmittel und 92,0 % (m/m) Wasser für Injektionszwecke als Dispersionsmedium mit einem Rotor-Stator-Dispergierer Ultra-Turrax T25 (Janke & Kunkel, Deutschland) bei 8.000 Umdrehungen pro Minute (UpM) für 15 Minuten und anschließend bei 13.500 UpM für eine Minute, fein dispergiert.

Die Partikelgröße der Beladungsflüssigkeit wurde bestimmt mittels eines Laserdiffraktometers (Mastersizer 2000, Malvern Instruments, Großbritannien) mit Auswertung nach der Fraunhofer Theorie. Unmittelbar nach Herstellung der Beladungssuspension lag die mittlere Größe (Durchmesser) von 50 % der Partikel bei 7,1 um (LD_{50%} 7,1 µm) und die mittlere Größe von 90 % der Partikel unter 39,3 µm (LD_{90%} 39,3 µm).

45 µl der milchigen leicht grünen bis gelblichen Mikrosuspension wurden direkt auf die Beladungsfläche pro Beladung homogen verteilt aufgetragen. Der gesamte Herstellungsprozess erfolgte unter Raumklima (T = 23.4 ± 1.3 °C; rF = 40 ± 2 %). Homogen beladene orale Filme mit fünfund zehnfacher Beladung (Fünferschritte) mit intermediärer Trocknung wurden hergestellt.

Der durchschnittliche Massenzuwachs des Wirkstoffs pro Beladung lag bei 0,19 mg/cm² und die relative Beladung bezogen auf das Gesamtgewicht des zehnfach beladenen oralen Films bei 13,4 % (m/m).

### Beispiel 11

Orale Filme hoch beladen mit Rutin wurden analog zu Beispiel 10 hergestellt.

Hingegen wurde im Vergleich zu Beispiel 10 eine Rutinnanosuspension genutzt, unter Anwendung einer abweichenden Herstellungsmethode unter Beibehaltung der qualitativen und quantitativen Zusammensetzung der finalen Rezeptur. Die Inhaltsstoffe wurden mit einer Kugelmühle PML-2 (Bühler AG, Schweiz) mit Mahlkugeln mit einer Größe zwischen 0,4-0,6 mm, bei 2.000 UpM, mit fünf Durchläufen, bei einer Temperatur von 5 °C, fein gemahlen. Das erhaltene Konzentrat wurde nach Entfernung der Mahlkugeln durch Verdünnung mit Wasser für Injektionszwecke auf die finale quantitative Konzentration verdünnt und durch Hochdruckhomogenisierung final fein dispergiert. Dazu wurde ein Hochdruckhomogenisierer Micron LAB 40 (APV Deutschland GmbH, Deutschland) unter Anwendung von 300 bar und zwei Durchläufen genutzt.

Unmittelbar nach Herstellung der Beladungssuspension lag ihre Partikelgröße bestimmt mit Laserdiffraktometrie LD_{50%} bei 0,240 µm und LD_{90%} bei 0,860 µm.

45 µl der milchigen leicht grünen bis gelblichen Nanosuspension wurden direkt auf die Beladungsfläche pro Beladung homogen verteilt aufgetragen. Verschiedene homogen beladene orale Filme mit fünf- bis fünfzigfacher Beladung (Fünferschritte) mit intermediäre Trocknung wurden hergestellt. Der durchschnittliche Massenzuwachs des Wirkstoffs pro Beladung lag bei 0,18 mg/cm² und die relative Beladung bezogen auf das Gesamtgewicht des fünfzigfach beladenen oralen Films lag bei 38,6 % (m/m).

### Beispiel 12

Orale Filme beladen mit Coenzym-Q10 wurden analog zu Beispiel 1 hergestellt.

Hingegen wurde im Vergleich zu Beispiel 1 eine stabile Nanoemulsion von Coenzym-Q10 als Beladungsmedium verwendet. Zu diesem Zweck wurde der Wirkstoff Coenzym-Q10 (Dr. Rimpler GmbH, Deutschland), in einer Konzentration von 2,5 % (m/m) in 5,0 % (m/m) mittelkettigen Triglyceriden (Caesar & Loretz GmbH, Deutschland) gelöst. Zusammen mit 2,5 % (m/m) Polysorbat 80 (VWR International GmbH, Deutschland) und Sorbitanmonooleat (Sigma-Aldrich Co. LLC., USA) als stabilisierende Tenside und 87,5 % (m/m) Wasser für Injektionszwecke als Dispersionsmedium mit einem Rotor-Stator-Dispergierer Ultra-Turrax T25 (Janke & Kunkel, Deutschland) bei 8.000 Umdrehungen pro Minute (UpM) für 30 Sekunden voremulgiert. Anschließend erfolgte die Feinemulgierung mittels Hochdruckhomogenisierung mit drei Durchläufen bei 500 bar und 85 °C mittels eines Hochdruckhomogenisierer Micron LAB 40 (APV Deutschland GmbH, Deutschland).

Unmittelbar nach Herstellung der Nanoemulsion lag ihre Partikelgröße bestimmt mit Laserdiffraktometrie LD_{50%} bei 0,136 um. Die Partikelgröße bestimmt mit Photonenkorrelationsspektroskopie mit einem Nanosizer ZS (Malvern Instruments Limited, Vereinigtes Königreich) lag bei 0,115 um und der Polydispersitätsindex bei 0,156.

90 µl der milchig cremigen leicht orangenen Nanoemulsion wurden direkt auf die Beladungsfläche pro Beladung homogen verteilt aufgetragen. Der gesamte Herstellungsprozess erfolgte unter Raumklima (T = 23,3 ± 0,9 °C; rF = 46 ± 8 %). Homogen beladene orale Filme mit fünfund zehnfacher Beladung (Fünferschritte) mit intermediärer Trocknung wurden hergestellt. Der durchschnittliche Massenzuwachs des Wirkstoffs pro Beladung lag bei 0,32 mg/cm² und die relative Beladung bezogen auf das Gesamtgewicht des zehnfach beladenen oralen Films bei 10,4 % (m/m).

### Beispiel 13

Orale Filme beladen mit Hesperidin wurden analog zu Beispiel 10 hergestellt.

Hingegen wurde im Vergleich zu Beispiel 10 anstatt Polysorbat 80, Poloxamer 188 (Kolliphor^{®} P188, BASF SE, Deutschland) als Stabilisator und Hesperidin (Denk Ingredients GmbH, Deutschland) als Wirkstoff eingesetzt.

Unmittelbar nach Herstellung der Beladungssuspension lag ihre Partikelgröße LD_{50%} bei 0,220 µm und LD_{90%} bei 0,700 um. Die Partikelgröße bestimmt mit Photonenkorrelationsspektroskopie lag bei 0,250 µm.

45 µl der milchigen grauen bis gelblichen Nanosuspension wurden direkt auf die Beladungsfläche pro Beladung homogen verteilt aufgetragen. Der gesamte Herstellungsprozess erfolgte unter Raumklima (T = 23.3 ± 0.9 °C; rF = 46 ± 8 %). Der durchschnittliche Massenzuwachs des Wirkstoffs pro Beladung lag bei 0,19 mg/cm² und die relative Beladung bezogen auf das Gesamtgewicht des zehnfach beladenen oralen Films bei 13,0 % (m/m).

### Beispiel 14

Orale Filme beladen mit Coenzym-Q10 wurden analog zu Beispiel 7 hergestellt.

Hingegen wurde im Vergleich zu Beispiel 7 eine Suspension von Coenzym-Q10 als Beladungsflüssigkeit verwendet. Zu diesem Zweck wurde der Wirkstoff Coenzym-Q10, in einer Konzentration von 2,0 % (m/m) mit 0,2 % (m/m) Poloxamer 188 (Kolliphor^{®} P188, BASF SE, Deutschland) als Stabilisator und 97,8 % (m/m) Wasser für Injektionszwecke als Dispersionsmittel mit einem Rotor-Stator-Dispergierer Ultra-Turrax bei 8.000 UpM für eine Minute vordispergiert. Anschließend erfolgte die Feindispergierung mittels Hochdruckhomogenisierung mit zwei Durchläufen bei 300 bar, dann mit zwei Durchläufen bei 500 bar, dann zwei Durchläufen bei 1.000 bar und abschließend zehn Durchläufen bei 1.500 bar.

Unmittelbar nach Herstellung der Beladungssuspension lag ihre Partikelgröße LD_{50%} bei 0,280 µm und LD_{90%} bei 0,850 um. Die Partikelgröße bestimmt mit Photonenkorrelations-spektroskopie lag bei 0,216 µm.

112,5 µl der orangenen bis gelblichen Nanosuspension wurden direkt auf die Beladungsfläche pro Beladung homogen verteilt aufgetragen. Der gesamte Herstellungsprozess erfolgte unter Raumklima (T = 23.3 ± 0.9 °C; rF = 46 ± 8 %). Der durchschnittliche Massenzuwachs des Wirkstoffs pro Beladung lag bei 0,18 mg/cm² und die relative Beladung bezogen auf das Gesamtgewicht des zehnfach beladenen oralen Films bei 12,6 % (m/m).

### Beispiel 15

Orale Filme beladen mit Folsäure wurden analog zu Beispiel 1 hergestellt.

Hingegen wurde im Vergleich zu Beispiel 1 der hergestellte orale Film mit einer Folsäure-Polymerdispersion überzogen. Dazu wurde Folsäure zu 0,25 % (m/m) in einer dreißig prozentigen wässrigen Ammoniummethacrylat-Copolymer-Dispersion (Typ A) (Eudragit RL 30D, Evonik Industries AG, Deutschland) zu 94,34 % (m/m) mit 5,66 % (m/m) Triacetin (HCH Highchem Hamburg GmbH, Deutschland) als Weichmacher verwendet. Der smartFilm wurde dabei mit so viel der Beladungsflüssigkeit überzogen, wie nötig war den smartFilm zu ummanteln. Nach Trocknung von Wasser als Dispersionsmedium besaß der orale Film eine durchgehende durchsichtige Polymermatrix mit eingebetteter Folsäure. Der gesamte Herstellungsprozess erfolgte unter Raumklima (T = 23,1 °C; rF = 61 %).

Der Massenzuwachs des Wirkstoffs für die einmalige Beladung lag bei 0,02 mg/cm² und die relative Beladung bezogen auf das Gesamtgewicht des zehnfach beladenen oralen Films bei 0,3 % (m/m).

### Beispiel 16

Orale Filme beladen mit Retinol wurden analog zu Beispiel 1 hergestellt.

Hingegen wurde im Vergleich zu Beispiel 1 eine wässrige stabile Lipidsuspension von Retinol als Beladungsflüssigkeit genutzt. Dazu wurde Retinol (BASF, Deutschland) in einer Konzentration von 4,8 % (m/m) in 14,45 % (m/m) geschmolzenem Cetylpalmitat und 0,75 % (m/m) mittelkettigen Triglyceriden gelöst. Die ölige Lösung wurde mit 1,8 % (m/m) Polyglyceryl-3-methylglucosedistearat (Tego Care 450) (Caesar & Loretz GmbH, Deutschland) als stabilisierendes Tensid in 78,2 % (m/m) Wasser für Injektionszwecke dispergiert. Eine Vordispersion wurde durch einen Rotor-Stator-Dispergierer Ultra-Turrax bei 8.000 UpM für 30 Sekunden geformt. Anschließend wurde diese Vordispersion durch Hochdruckhomogenisierung mit drei Durchläufen bei 500 bar und 85 °C fein dispergiert.

Nach Herstellung der Beladungssuspension lag ihre Partikelgröße LD_{50%} bei 0,127 µm. Die Partikelgröße bestimmt mit Photonenkorrelationsspektroskopie lag bei 0,106 µm und der Polydispersitätsindex bei 0,121.

46,9 µl der milchig gelblichen Nanolipidsuspension wurden direkt auf die Beladungsfläche pro Beladung homogen verteilt aufgetragen. Der gesamte Herstellungsprozess erfolgte unter Raumklima (T = 23.3 ± 0.9 °C; rF = 46 ± 8 %).

Der durchschnittliche Massenzuwachs des Wirkstoffs pro Beladung lag bei 0,13 mg/cm² und die relative Beladung bezogen auf das Gesamtgewicht des zehnfach beladenen oralen Films bei 7,5 % (m/m).

### Beispiel 17

Orale Filme beladen mit Coenzym-Q10 wurden analog zu Beispiel 16 hergestellt.

Hingegen wurde im Vergleich zu Beispiel 16 eine wässrige stabile orangefarbene Lipidsuspension von Coenzym-Q10 als Beladungsflüssigkeit genutzt. Dazu wurde Coenzym-Q10 (Dr. Rimpler GmbH, Deutschland) als Reinstoff in einer Konzentration von 4,8 % (m/m) in 14,45 % (m/m) geschmolzenem Cetylpalmitat und 0,75 % (m/m) mittelkettigen Triglyceriden gelöst. Die ölige Lösung wurde mit 1,8 % (m/m) Polyglyceryl-3-methylglucosedistearat (Tego Care 450) (Caesar & Loretz GmbH, Deutschland) als stabilisierendes Tensid in 78,2 % (m/m) Wasser für Injektionszwecke dispergiert. Eine Vordispersion wurde durch einen Rotor-Stator-Dispergierer Ultra-Turrax bei 8.000 UpM für 30 Sekunden geformt. Anschließend wurde diese Vordispersion durch Hochdruckhomogenisierung mit drei Durchläufen bei 500 bar und 85 °C fein dispergiert.

Nach Herstellung der Beladungslipidsuspension lag ihre Partikelgröße LD_{50%} bei 0,223 µm. Die Partikelgröße bestimmt mit Photonenkorrelationsspektroskopie lag bei 0,214 um und der Polydispersitätsindex lag bei 0,124. Der gesamte Herstellungsprozess erfolgte unter Raumklima (T = 25.2 ± 0.9 °C; rF = 43 ± 3 %).

Der durchschnittliche Massenzuwachs des Wirkstoffs pro Beladung lag bei 0,21 mg/cm² und die relative Beladung bezogen auf das Gesamtgewicht des zehnfach beladenen oralen Films bei 9,0 % (m/m).

### Beispiel 18

Orale Filme beladen mit Coenzym-Q10 wurden analog zu Beispiel 17 hergestellt.

Hingegen wurde im Vergleich zu Beispiel 17 das stabilisierende Tensid Polyglyceryl-3-methylglucosedistearat gegen Poloxamer 188 (Kolliphor^{®} P188, BASF SE, Deutschland) ersetzt.

Nach Herstellung der Beladungslipidsuspension lag ihre Partikelgröße LD_{50%} bei 0,148 um. Die Partikelgröße bestimmt mit Photonenkorrelationsspektroskopie lag bei 0,252 um und der Polydispersitätsindex lag bei 0,192.

Der durchschnittliche Massenzuwachs des Wirkstoffs pro Beladung lag bei 0,13 mg/cm² und die relative Beladung bezogen auf das Gesamtgewicht des zehnfach beladenen oralen Films bei 6,53 % (m/m).

### Beispiel 19

Orale Filme beladen mit Coenzym-Q10 wurden analog zu Beispiel 17 hergestellt.

Hingegen wurde im Vergleich zu Beispiel 17 das stabilisierende Tensid Polyglyceryl-3-methylglucosedistearat gegen Cetylpyridiniumchlorid ersetzt. Durch die Verwendung von Cetylpyridiniumchlorid erhielten die Partikel eine positive Ladung und dadurch bedingt eine erhöhte Adsorptionsneigung an die negativ geladenen Cellulosefasern.

Nach Herstellung der Beladungslipidsuspension lag ihre Partikelgröße LD_{50%} bei 0,310 um. Die Partikelgröße bestimmt mit Photonenkorrelationsspektroskopie lag bei 0,083 µm und der Polydispersitätsindex lag bei 0,124.

Der durchschnittliche Massenzuwachs des Wirkstoffs pro Beladung lag bei 0,19 mgcm² und die relative Beladung bezogen auf das Gesamtgewicht des zehnfach beladenen oralen Films bei 8,4 % (m/m).

### Beispiel 20

Die Beladung der Inhaltsstoffe insbesondere der Wirkstoffe der oralen Filme der Beispiele 1-19 wurde gravimetrisch bestimmt. Dadurch konnte sowohl der durchschnittliche Massenzuwachs pro Beladung als auch die relative Beladungskonzentration der einzelnen Beispiele bestimmt werden.

Dazu wurde der jeweilig zu untersuchende orale Film mittels einer Analysenwaage BP210S (Sartorius, Deutschland) mit einer Ablesegenauigkeit d = 0,1 mg, unter den gleichen klimatischen Bedingungen wie bei Beladung, nach Trocknung gewogen. Zur Sicherung der abgeschlossenen Trocknung des oralen Films wurde ein gravimetrisch bestimmtes Ergebnis nur nach vorheriger Massekonstanz (≤ 0,5 mg) zweier nacheinander folgender Wägungen nach einem Tag akzeptiert. War keine Massenkonstanz eingetreten, so wurde ein weiterer Trocknungstag eingelegt. Zur Wiegung wurden pro Beispiel verschiedenen Größen oraler Filme an verschiedenen Beladungen herangezogen. Durch lineare Regression der gewogenen Masse verschiedener Beladungen erfolgte die Ermittlung des Wirkstoffzuwachses pro Beladung anhand des Anstiegs der Regressionsgeraden.

### Beispiel 21

Die Dicke der oralen Filme der Beispiele 1 und 11 wurde bestimmt.

Die Dicke als Maß für die Homogenität der Beladung über die gesamte Fläche des oralen Films wurde mittels einer Mikrometerschraube mit einer Ablesegenauigkeit d = 0,01 mm bestimmt. Dazu wurden jeweils drei orale Filme mit einer Größe von 4 cm² (2 x 2 cm) an verschiedenen Beladungsstufen (Fünferschritte) an allen vier Ecken und der in der Mitte vermessen, um die gesamte Probenfläche repräsentativ abzubilden. Die Dicke wurde dabei so abgenommen, dass es zu keiner Veränderung des oralen Films kam.

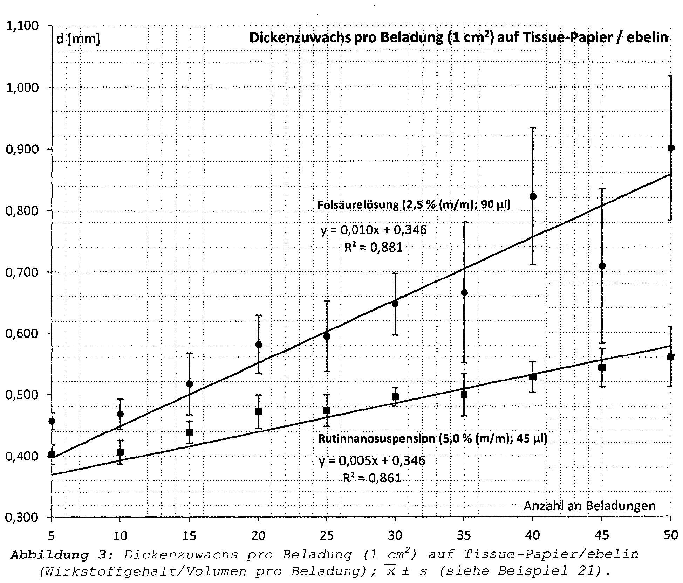

In Abbildung 3 ist die Anzahl der Beladung (Abszissenachse) gegen die Dicke in mm (Ordinatenachse) aufgetragen. Mit steigender Wirkstoffkonzentration bzw. Beladungsanzahl steigt auch die Dicke der oralen Filme in linearer Korrelation. Der Schnittpunkt der linearen Regressionsgeraden mit der Ordinatenachse bildet die Dicke des nicht beladenen smartFilms ab. Die in Abbildung 3 verdeutlichte Standardabweichung in Form von positiven und negativen Fehlerbalken ist ein Maß für die Homogenität der Wirkstoffbeladung. Bei gleicher aufgetragener Masse wurde mit der Rutinnanosuspension eine homogenere Wirkstoffbeladung erzielt als mit der Folsäurelösung.

### Beispiel 22

Röntgendiffraktogramme der fünfzigfach beladenen der oralen Filme des Beispiels 1 mit Wirkstoff Folsäure wurden nach Herstellung, nach drei, sechs, neun und zwölf Monaten aufgenommen.

Die Beladungsseite des jeweiligen oralen Films wurde mit Röntgenstrahlen aus einem Röntgengenerator PW 1830 (Philips, Niederlande) bei einer Spannung von 40 kV und einer Stromstärke von 25 mA, bestrahlt. Die Wellenlänge des Lichtes betrug 1,5418 Å. Die oralen Filme wurden in Schritten von 0,04 ° in einem Winkel von 0,6 ° bis 40 ° abgetastet.

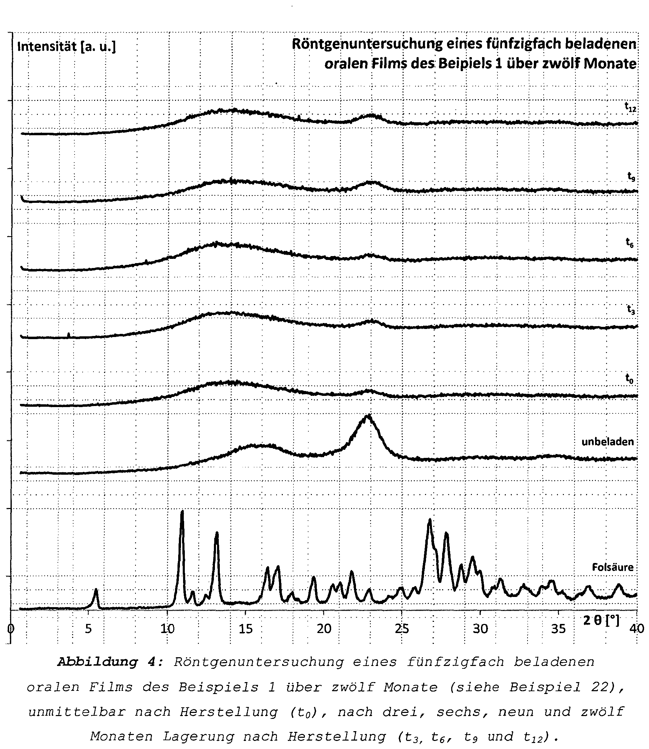

In Abbildung 4 ist ersichtlich, dass die Diffraktogramme der beladenen oralen Filme (fünfzigfache Beladung) sich zu keinem der untersuchten Zeitpunkte nach der Herstellung, nach drei, sechs, neun oder zwölf Monaten von denen des unbeladenen smartFilms unterscheiden. Es sind keine Spitzen wie beim Folsäure erkennbar, welcher als Reinstoff kristallin vorliegt. Zu keinem Zeitpunkt innerhalb von mindestens zwölf Monaten liegt der Wirkstoff in den oralen Filmen kristallin vor.

### Beispiel 23

Rasterelektronenmikroskopieaufnahmen der smartFilms wurden aufgenommen.

Zur Herstellung der Leitfähigkeit der Proben erfolgte ein Überziehen mit einer dünnen Schicht aus Gold und Palladium mit dem Sputtering System SCD 40 (Balzers Union, Deutschland). Die leitfähigkeitsgewährleistende Schicht ist dabei so dick gewählt, dass es zu keiner irreversiblen Veränderung durch Artefaktbildung während der Aufnahmen kam. Die beschichteten smartFilms wurden auf einem Aluminiumstiftprobenteller in das Rasterelektronenmikroskop DSM 950 (Zeiss, Deutschland) integriert und bei einer Spannung von 10,0 kV unter Vakuum bebildert. Die Detektion erfolgte mittels Sekundärelektronen bei verschiedenen Arbeitsabständen.

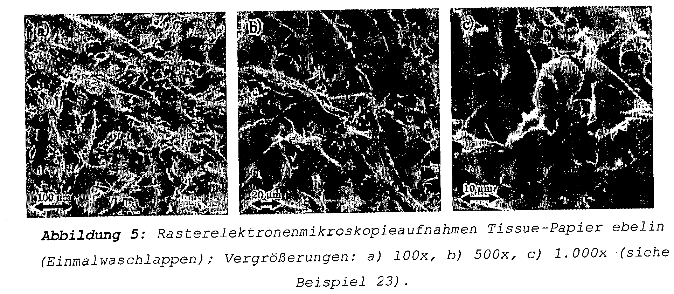

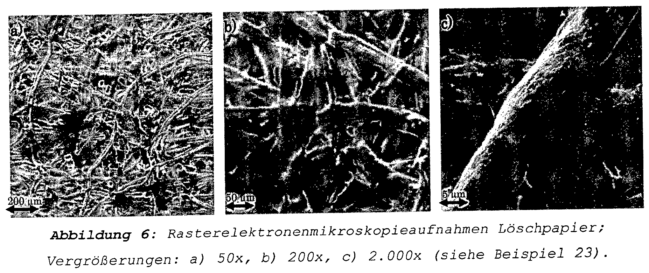

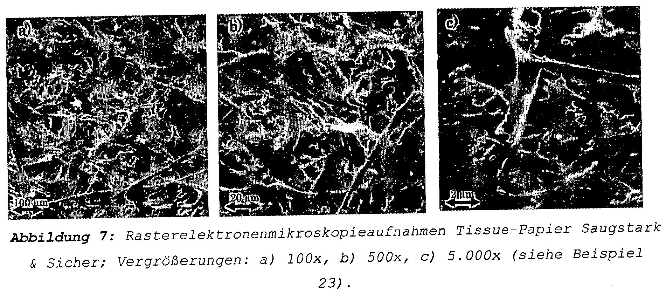

Verschiedene Vergrößerungen wurden zur idealen Veranschaulichung der smartFilms aufgenommen (siehe Abbildung 5, 6 und 7) .

Anhand je einer repräsentativen Aufnahme der Rasterelektronenmikroskopie der smartFilms wurden die Porendurchmesser bestimmt. Dazu wurden alle auf der Oberfläche erkennbaren Poren kreisförmig normiert und der Durchmesser ausgemessen. Die Porendurchmesser waren bei allen drei smartFilms polydispers verteilt wobei der mittlere Porendurchmesser bei 21 µm für die Einmalwaschlappen, 57 µm für das Löschpapier und bei 50 µm für die Küchentücher lag. Die maximalen Porendurchmesser lagen bei 205 µm für die Einmalwaschlappen, 235 µm für das Löschpapier und bei 171 µm für die Küchentücher. Die minimalen Porendurchmesser lagen bei 7 um für die Einmalwaschlappen, 29 µm für das Löschpapier und bei 14 µm für die Küchentücher.

### Beispiel 24

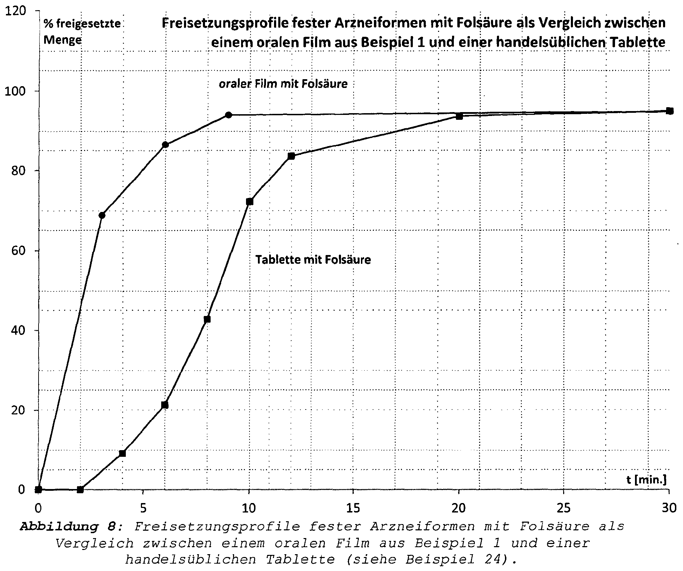

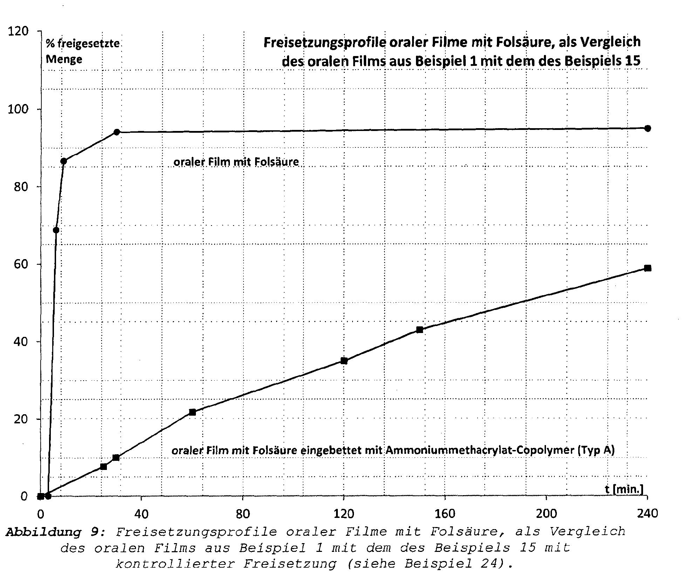

Ein Wirkstofffreisetzungsprofil des oralen Films des Beispiels 1 (fünfzigfach beladen) wurde vergleichend zu dem Wirkstofffreisetzungsprofil des oralen Films des Beispiels 15 (siehe Abbildung 9) aufgenommen. Des Weiteren wurde ein Wirkstofffreisetzungsprofil des oralen Films des Beispiels 1 (fünfzigfach beladen) vergleichend zu dem Wirkstofffreisetzungsprofil mit den kommerziell erhältlichen Tabletten des Produktes Folsäure STADAO 5 mg (STADA AG, Deutschland) (siehe Abbildung 8) aufgenommen.

Dazu wurde eine Freisetzungsapparatur PTW SIII (Pharmatest, Deutschland) mit einem Blattrührer, gemäß Ph. Eur. und einem Beschwerer (Metallkorb, V = 20 cm³), welcher die zu untersuchende Applikationsform kontrolliert an einer definierten Stelle am Boden des normierten Becherglases hält, verwendet. Die Blattrührergeschwindigkeit lag bei 100 UpM. Als Freisetzungsgefäß diente ein normiertes Becherglas, gemäß Ph. Eur. aus Braunglas mit Deckel, gegen eventuelle Verdunstungsverluste. Die Temperatur des Freisetzungsmediums wurde mittels eines Thermostats konstant auf 37 ± 0.5 °C Solltemperatur gehalten und diese mit einem externen Thermometer routinemäßig in regelmäßigen Abständen geprüft. Als Freisetzungsmedium dienten 1.000 ml einer wässrigen Phosphatpufferlösung mit einem pH von 6,8 ± 0,05. Direkt nach dem Probenzug wurden die entsprechenden Proben mittels UV-Vis-Spektroskopie mit einem Zweistrahlspektrophotometer Pharma Spec UV-1700 (Shimadzu, Japan) im Absorptionsmaximum des Wirkstoffs Folsäure bei 0,283 µm vermessen.

In Abbildung 8 ist ersichtlich, dass die im Beispiel 1 hergestellten oralen Filme den gleichen Wirkstoff schneller freigeben (schnelleres Erreichen des Maximums mit höherem Anstieg) als eine handelsübliche Tablette als gängigste orale Applikationsform. Die Freisetzung des oralen Films beginnt sofort durch Auswaschung aus dem smartFilm, wohingegen die Tablette erste zerfallen muss um anschließend den Wirkstoff freizusetzen. Dies stellt eine Beschleunigung der Freisetzung im Vergleich zu bisherigen oralen Applikationsformen dar, welche erst zerfallen müssen, um die Inhaltsstoffe insbesondere die Wirkstoffe freizusetzen.

In Abbildung 9 ist zu erkennen, dass mit den erfindungsgemäßen smartFilms und dem Herstellungsverfahren Filmprodukte zur oralen und peroralen Applikation hergestellt werden können, welche den Wirkstoff kontrolliert freisetzen. Hier durch das Beispiel 15 mit einer Kinetik 0. Ordnung beschrieben. Neben dem hier beschriebenen Freisetzungsprofil sind beispielsweise auch verlängerte, verzögerte und/oder gesteuerte (nach einer gewünschten Kinetik) Freisetzungsprofile möglich ohne auf diese beschränkt zu sein.

### Beispiel 25

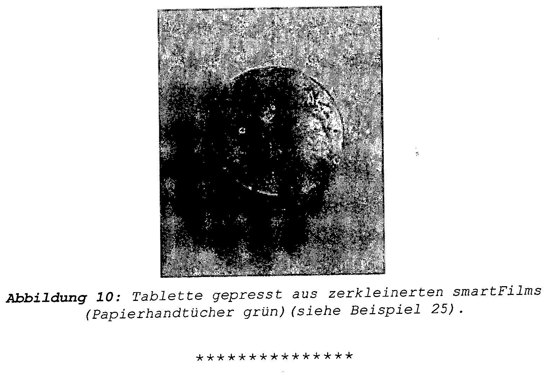

Toiletten-Papierhandtücher (grün) wurden zu einer Tablette gepresst. Das Papier wurde dazu grob zerrissen und in die Matrizenöffnung einer Excentertablettenmaschine Exacta (Fette Compacting GmbH, Deutschland) gefüllt, und mit einem Spatel leicht zusammen gepresst. Anschließend erfolgte manuelles Durchdrehen der Maschine über die Excenterscheibe und Komprimieren der smart-Film-Stücke zu einer festen Tablette (siehe Abbildung 10).

Es folgen zehn Abbildungen.

## Patentansprüche

1. Stabile, physiologisch cellulosefaserbasierte Trägermatrix auf Papierbasis (smartFilm) zur Herstellung von Filmprodukten zur oralen und peroralen Applikation, **dadurch gekennzeichnet, dass** diese sich aus flächiger bis cuboider faserförmiger Cellulose in Form eines beständigen Papiermaterials aus ein oder mehreren Lagen mit einem Kristallinitätsindex von 1 ≤ Krl ≤ 200 bevorzugt zwischen 18 und 70, einer scheinbaren Blattdichte von 0,10 ≤ ρ ≤ 2,00 g/cm³ bevorzugt zwischen 0,36 und 0,51 g/cm³, einem Wasseraufnahmevermögen von 10 ≤ M_{Wasseraufnahme} ≤ 2.000 % (m/m) bevorzugt zwischen 195 und 388 % (m/m), einem durchschnittlichen Porendurchmesser von 1 ≤ d_{Pore} 200 µm bevorzugt zwischen 21 und 57 µm und einem Wassergehalt von 1,0 ≤ w ≤ 20,0 % (m/m) bevorzugt zwischen 7,5 und 9,5 % (m/m) beladen mit Inhaltsstoffen insbesondere Wirkstoffen zusammensetzt.

2. Stabile, physiologisch geeignete cellulosefaserbasierte Trägermatrix auf Papierbasis (smartFilm) zur Herstellung von Filmprodukten zur oralen und peroralen Applikation nach Anspruch 1, **dadurch gekennzeichnet, dass** die Inhaltsstoffe insbesondere der oder die Wirkstoff(e) eine relative Beladung bezogen auf das Gesamtgewicht von 0,1 ≤ β_{Beladung} ≤ 99,9 % vorzugweise hochbeladen ≥ 70 % eingebettet in das Porenvolumen und auf der Oberfläche adsorbiert vorliegen.

3. Stabile, physiologisch geeignete cellulosefaserbasierte Trägermatrix auf Papierbasis (smartFilm) zur Herstellung von Filmprodukten zur oralen und peroralen Applikation nach Anspruch 1, **dadurch gekennzeichnet, dass** diese eine geometrische Form besitzt beispielsweise eine quadratische, rechteckige, runde oder ovale Form hat und als Applikationsform oraler Film eine Fläche von 0,1 ≤ A_{Filmprodukt} ≤ 9 cm², vorzugsweise 1-4 cm² besitzt.

4. Stabile, physiologisch geeignete cellulosefaserbasierte Trägermatrix auf Papierbasis (smartFilm) zur Herstellung von Filmprodukten zur oralen und peroralen Applikation nach Anspruch 1 bis 3, **dadurch gekennzeichnet, dass** auf dieser verschiedene Inhaltsstoffe insbesondere Wirkstoff(e), beispielsweise nichtopiode Analgetika (z. B. Acetylsalicylsäure, Paracetamol, Ibuprofen), opioide Analgetika (z. B. Tilidin, Codein, Fentanyl), Antibiotika (z. B. Clarithromycin, Clindamycin, Minocylin), Betarezeptorenblocker (z. B. Metoprolol, Bisoprolol, Nebivolol), AT₁-Rezeptorantagonisten (z. B. Valsartan, Losartan, Candesartan), Angiotensinkonversionsenzym-Hemmer (z. B. Ramipril, Captopril, Enalapril), Diuretika (z. B. Furosemid, Hydrochlorothiazid, Spironolacton), Calciumkanalblocker (z. B. Nifedipin, Diltiazem, Verapamil), Protonenpumpenhemmer (z. B. Pantoprazol, Omeprazol, Lansoprazol), HMG-CoA-Reduktasehemmer (Simvastatin, Lovastatin, Pravastatin), Schilddrüsenhormone (z. B. L-Thyroxin), Glucocorticoide (z. B. Prednisolon, Prednison, Dexamethason), Antidepressiva (z. B. Amitryptilin, Mianserin, Fluoxetin), Neuroleptika (z. B. Clozapin, Haloperidol, Perazin), Benzodiazepine (z. B. Diazepam, Tetrazepam, Midazolam), Benzodiazepinanaloga (z. B. Zaleplon, Zopiclon, Zolpidem), Antihistaminika (z. B. Doxylamin, Cetirizin, Ranitidin, Famotidin), Lokalanästhetika (z. B. Lidocain, Tetracain, Mepivacain), Antiepileptika (z. B. Carbamazepin, Phenytoin, Valproinsäure), Antiparkinsonika (z. B. Levodopa, Benserazid, Carbidopa), Antidiabetika (z. B. Metformin, Glibenclamid, Rosiglitazon, Sitagliptin, Repaglinid) und Thrombozytenaggregationshemmer (z. B. Ticlopidin, Clopidogrel) getrennt oder zusammen aufgebracht sind.

5. Stabile, physiologisch geeignete cellulosefaserbasierte Trägermatrix auf Papierbasis (smartFilm) zur Herstellung von Filmprodukten zur oralen und peroralen Applikation nach Anspruch 1 bis 4, **dadurch gekennzeichnet, dass** die Wirkstofffreisetzung im gesamten Verdauungstrakt von der Mundhöhle bis zum Rektum durch die Beladung mit speziellen Hilfsstoffen beispielsweise Gelatine, Polyvinylpyrrolidon oder Hydroxypropylmethylcellulose verlängert, verzögert, beschleunigt und/oder gesteuert wird.

6. Stabile, physiologisch geeignete cellulosefaserbasierte Trägermatrix auf Papierbasis (smartFilm) nach Anspruch 1 und 3, **dadurch gekennzeichnet, dass** diese nicht zerfallen muss, um die Inhaltsstoffe freizusetzen und dadurch die Freisetzungszeit nicht durch die Zerfallszeit der Applikationsform verlängert wird, wodurch es zu einer stoßartigen sofortigen Freisetzung (engl. burst release) der Inhaltsstoffe bedingt durch Auswaschung nach Kontakt mit den Verdauungsflüssigkeiten kommt.

7. Stabile, physiologisch geeignete cellulosefaserbasierte Trägermatrix auf Papierbasis (smartFilm) zur Herstellung von Filmprodukten zur oralen und peroralen Applikation nach Anspruch 1 bis 4, **dadurch gekennzeichnet, dass** neben Wirkstoffen auch Farbstoffe, Aromen, Süßungsmittel, Speichelstimulanzien, Wirkstoffstabilisatoren und Verfahrenshilfsstoffe, ohne Weichmacher oder Trägerstabilisatoren, getrennt oder zusammen vorhanden sind.

8. Stabile, physiologisch geeignete cellulosefaserbasierte Trägermatrix auf Papierbasis (smartFilm) zur Herstellung von Filmprodukten zur oralen und peroralen Applikation nach Anspruch 1 bis 4 und 7, **dadurch gekennzeichnet, dass** sie beladen ist mit Nanopartikeln, vorzugsweise aufgebracht als Suspension, d. h. Nanopartikel dispergiert in einem flüssigen wässrigen oder organischen Dispersionsmedium, und optional anschließender Entfernung des Dispersionsmediums, wobei die Nanopartikel sind insbesondere Polymernanopartikel, Liposomen, Cubosomen, Nanoemulson, Lipidnanopartikel (solid lipid nanoparticles - SLN, nanostructured lipid carriers - NLC, smartLipids) und Mizellen aus Tensiden und Polymeren.

9. Stabile, physiologisch geeignete cellulosefaserbasierte Trägermatrix auf Papierbasis (smartFilm) zur Herstellung von Filmprodukten zur oralen und peroralen Applikation nach Anspruch 1 bis 4 und 7, **dadurch gekennzeichnet, dass** sie beladen ist mit Nanopartikeln in Form von Nanokristallen, vorzugsweise aufgebracht als Nanosuspension, d. h. Nanokristalle dispergiert in einem flüssigen wässrigen oder organischen Dispersionsmedium, und optional anschließende Entfernung des Dispersionsmediums.

10. Stabile, physiologisch geeignete cellulosefaserbasierte Trägermatrix auf Papierbasis (smartFilm) zur Herstellung von Filmprodukten zur oralen und peroralen Applikation nach Anspruch 1 bis 4 und 7, **dadurch gekennzeichnet, dass** sie beladen ist mit einer oder mehreren Mikroemulsionen oder einem oder mehreren selbstemulgierenden Systemen, vorzugsweise aufgebracht in flüssiger Form oder als Präkonzentrat, welches in Kontakt mit Wasser eine Mikroemulsion oder ein selbstemulgierendes System bildet.

11. Stabile, physiologisch geeignete cellulosefaserbasierte Trägermatrix auf Papierbasis (smartFilm) zur Herstellung von Filmprodukten zur oralen und peroralen Applikation nach Anspruch 1 bis 4 und 7, **dadurch gekennzeichnet, dass** sie beladen ist mit Mikropartikeln, vorzugsweise aufgebracht als Suspension, d. h. Mikropartikel dispergiert in einem flüssigen wässrigen oder organischen Dispersionsmedium, und optional anschließender Entfernung des Dispersionsmediums. Mikropartikel besitzen eine mittlere Partikelgröße (Durchmesser 50%) von 1 µm bis < 1.000 µm, wobei die Mikropartikel sind insbesondere Wirkstoffkristalle, organische Mikropartikel (z. B. Cellulosepartikel), Polymerpartikel, Liposomen, Emulsionen, Lipidmikropartikel und anorganische Mikropartikel (z. B. Silica).

12. Stabile, physiologisch geeignete cellulosefaserbasierte Trägermatrix auf Papierbasis (smartFilm) zur Herstellung von Filmprodukten zur oralen und peroralen Applikation nach Anspruch 1 bis 4 und 7, **dadurch gekennzeichnet, dass** sie beladen ist mit porösen Mikropartikeln, vorzugsweise aufgebracht als Suspension, d. h. Mikropartikel dispergiert in einem flüssigen wässrigen oder organischen Dispersionsmedium, oder in flüssigem Kohlendioxid, und optional anschließender Entfernung des Dispersionsmediums, wobei bevorzugte Materialien für poröse Mikropartikel sind Cellulosen, Silikate oder Calciumcarbonat und die porösen Partikel sind beladen mit Wirkstoff oder Wirkstoffen.

13. Stabile, physiologisch geeignete cellulosefaserbasierte Trägermatrix auf Papierbasis (smartFilm) zur Herstellung von Filmprodukten zur oralen und peroralen Applikation nach Anspruch 1-4 und 7, **dadurch gekennzeichnet, dass** enthaltene Inhaltsstoffe, Wirkstoffe und/oder Partikel mittels 3D-Druck mittels eines 3D-Druckers aufgebracht werden, und dabei optional bestimmte Anordnungen der Stoffe und dreidimensionale Formen erzeugt werden.

14. Filmprodukte zur oralen und peroralen Applikation hergestellt aus stabilen, physiologisch geeigneten cellulosefaserbasierten Trägermatrices auf Papierbasis (smartFilms) nach Anspruch 1, 2 und 4 bis 12, **dadurch gekennzeichnet, dass** diese als Zwischenprodukte in ihrer Größe reduziert und in ihrer Form verändert werden, beispielsweise durch Schneiden oder Häckseln und anschließend in andere feste (z. B. Tabletten, Kapseln oder Kaugummis), halbfeste (z. B. Gele, Salben oder Kaugummis) oder flüssige Applikationssysteme (z. B. Lösungen oder Suspensionen) weiterverarbeitet werden, wobei die charakteristischen Freisetzungseigenschaften der Filmprodukte hergestellt aus smartFilms werden zusammen mit der Konservierung des kristallinen Zustands der Inhaltsstoffe an die jeweilige Applikationsform weitergegeben.

15. Verfahren zur Herstellung von Filmprodukten zur oralen und peroralen Applikation aus stabilen, physiologisch geeigneten cellulosefaserbasierte Trägermatrix auf Papierbasis (smartFilm) nach Anspruch 1-4 und 7-13, **dadurch gekennzeichnet, dass** durch ein mehrstufiges Beladungsverfahren mit intermediärer Trocknung die flächige faserförmige Cellulose als Film mit beliebigen Wirkstoffen und Hilfsstoffen, in beliebiger Menge, mit Flüssigkeiten in Form von Lösungen, Suspension oder Emulsionen, in verschiedenen Teilchengrößen, zu verschiedenen Zeiten, beladen werden kann, wobei flüssige Dispersionmedien beispielsweise Wasser, Ethanol, Propylenglykol, flüssige Polyethylenglykole, Glycerol, fette Öle sowie deren Mischungen sein können.

16. Verfahren zur Herstellung von Filmprodukten zur oralen und peroralen Applikation aus stabilen, physiologisch geeigneten cellulosefaserbasierte Trägermatrix auf Papierbasis (smartFilm) nach Anspruch 1-4 und 7-13, **dadurch gekennzeichnet, dass** das Beladungsverfahren nach Abschluss des Filmformungsschritt getrennt erfolgt, ohne thermische Belastung des oder der Inhaltsstoffe insbesondere der Wirkstoffe und ohne Störung des Filmformungsschrittes erfolgt.

## Claims

1. Stable, physiologically cellulose fibre-based carrier matrix on a paper basis (smartFilm) for the production of film products for oral and peroral application, **characterised, in that** it is comprised of laminar to cuboid fibrous cellulose in the form of a stable paper material comprising one or more layers with a crystallinity index of 1 ≤ Krl ≤ 200 preferably between 18 and 70, an apparent sheet density of 0.10 ≤ ρ ≤ 2.00 g/cm³ preferably between 0.36 and 0.51 g/cm³, a water absorption capacity of 10 ≤ M_{water absorption} ≤ 2,000 % (m/m) preferably between 195 and 388 % (m/m), an average pore diameter of 1 ≤ dₚₒᵣₑ 200 µm preferably between 21 and 57 µm and a water content of 1.0 ≤ w ≤ 20.0 % (m/m) preferably between 7.5 and 9.5 % (m/m) loaded with ingredients, in particular active ingredients.

2. Stable, physiologically suitable cellulose fibre-based carrier matrix on a paper basis (smartFilm) for the production of film products for oral and peroral application according to claim 1, **characterised in that** the ingredients, in particular the active ingredient or the active ingredients, have a relative loading relative to the total weight of 0.1 ≤ P_{load} ≤ 99.9 %, preferably highly loaded ≥ 70 %, embedded in the pore volume and adsorbed on the surface.

3. Stable, physiologically suitable cellulose fibre-based carrier matrix on a paper basis (smartFilm) for the production of film products for oral and peroral application according to claim 1, **characterised in that** it has a geometric shape, for example a square, rectangular, round or oval shape, and has an area of 0.1 ≤ A_{film product} ≤ 9 cm², preferably 1-4 cm², as the application form of oral film.

4. Stable, physiologically suitable cellulose fibre-based carrier matrix on a paper basis (smartFilm) for the production of film products for oral and peroral application according to claims 1 to 3, **characterised in that** different ingredients, in particular active ingredient(s), for example non-opioid analgesics (e.g. acetylsalicylic acid, paracetamol, ibuprofen), opioid analgesics (e.g. tilidine, codeine, fentanyl), antibiotics (e.g. clarithromycin, clindamycin, minocyline), beta-receptor blockers (e.g. metoprolol, bisoprolol, nebivolol), AT₁ receptor antagonists (e.g. valsartan, losartan, candesartan), angiotensin-converting enzyme inhibitors (e.g. ramipril, captopril, enalapril), diuretics (e.g. furosemide, hydrochlorothiazide, spironolactone), calcium channel blockers (e.g. nifedipine, diltiazem, verapamil), proton pump inhibitors (e.g. pantoprazole, omeprazole, lansoprazole), HMG-CoA reductase inhibitors (simvastatin, lovastatin, pravastatin), thyroid hormones (e.g. L-thyroxine), glucocorticoids (e.g. prednisolone, prednisone, dexamethasone), antidepressants (e.g. amitryptiline, mianserin, fluoxetine), neuroleptics (e.g. clozapine, haloperidol, perazine), benzodiazepines (e.g. diazepam, tetrazepam, midazolam), benzodiazepine analogues (e.g. zaleplon, zopiclone, zolpidem), antihistamines (e.g. doxylamine, cetirizine, ranitidine, famotidine), local anaesthetics (e.g. lidocaine, tetracaine, mepivacaine), antiepileptic drugs (e.g. carbamazepine, phenytoin, valproic acid), anti parkinsonian drugs (e.g. levodopa, benserazide, carbidopa), antidiabetics (e.g. metformin, glibenclamide, rosiglitazone, sitagliptin, repaglinide) and platelet aggregation inhibitor (e.g. ticlopidine, clopidogrel) are applied separately or together on it.

5. Stable, physiologically suitable cellulose fibre-based carrier matrix on a paper basis (smartFilm) for the production of film products for oral and peroral application according to claims 1 to 4, **characterised in that** the release of active ingredient in the entire digestive tract from the oral cavity to the rectum is extended, delayed, accelerated and/or controlled by loading with special excipients, for example gelatine, polyvinylpyrrolidone or hydroxypropylmethylcellulose.

6. Stable, physiologically suitable cellulose fibre-based carrier matrix on a paper basis (smartFilm) according to claims 1 and 3, **characterised in that** it does not have to disintegrate in order to release the ingredients and the release time is thus not prolonged by the disintegration time of the application form, resulting in an immediate burst release of the ingredients due to leaching after contact with the digestive fluids.

7. Stable, physiologically suitable cellulose fibre-based carrier matrix on a paper basis (smartFilm) for the production of film products for oral and peroral application according to claims 1 to 4, **characterised in that**, in addition to active ingredients, colourings, flavourings, sweeteners, saliva stimulants, active ingredient stabilisers and processing excipients, without plasticisers or carrier stabilisers, are present separately or together.

8. Stable, physiologically suitable cellulose fibre-based carrier matrix on a paper basis (smartFilm) for the production of film products for oral and peroral application according to claims 1 to 4 and 7, **characterised in that** it is loaded with nanoparticles, preferably applied as a suspension, i.e. nanoparticles dispersed in a liquid aqueous or organic dispersion medium, and optionally subsequent removal of the dispersion medium, wherein the nanoparticles are in particular polymer nanoparticles, liposomes, cubosomes, nanoemulsions, lipid nanoparticles (solid lipid nanoparticles - SLN, nanostructured lipid carriers - NLC, smart lipids) and micelles of surfactants and polymers.

9. Stable, physiologically suitable cellulose fibre-based carrier matrix on a paper basis (smartFilm) for the production of film products for oral and peroral application according to claims 1 to 4 and 7, **characterised in that** it is loaded with nanoparticles in the form of nanocrystals, preferably applied as a nanosuspension, i.e. nanocrystals dispersed in a liquid aqueous or organic dispersion medium, and optionally subsequent removal of the dispersion medium.

10. Stable, physiologically suitable cellulose fibre-based carrier matrix on a paper basis (smartFilm) for the production of film products for oral and peroral application according to claims 1 to 4 and 7, **characterised in that** it is loaded with one or more microemulsions or one or more self-emulsifying systems, preferably applied in liquid form or as a preconcentrate, which forms a microemulsion or a self-emulsifying system in contact with water.

11. Stable, physiologically suitable cellulose fibre-based carrier matrix on a paper basis (smartFilm) for the production of film products for oral and peroral application according to claims 1 to 4 and 7, **characterised in that** it is loaded with microparticles, preferably applied as a suspension, i.e. microparticles dispersed in a liquid aqueous or organic dispersion medium, and optionally subsequent removal of the dispersion medium. Microparticles have a mean particle size (diameter 50%) of 1 µm to < 1,000 µm, the microparticles being in particular active ingredient crystals, organic microparticles (e.g. cellulose particles), polymer particles, liposomes, emulsions, lipid microparticles and inorganic microparticles (e.g. silica).

12. Stable, physiologically suitable cellulose fibre-based carrier matrix on a paper basis (smartFilm) for the production of film products for oral and peroral application according to claims 1 to 4 and 7, **characterised in that** it is loaded with porous microparticles, preferably applied as a suspension, i.e. microparticles dispersed in a liquid aqueous or organic dispersion medium, or in liquid carbon dioxide, and optionally subsequent removal of the dispersion medium, wherein preferred materials for porous microparticles are celluloses, silicates or calcium carbonate, and the porous particles are loaded with active ingredient or active ingredients.

13. Stable, physiologically suitable cellulose fibre-based carrier matrix on a paper basis (smartFilm) for the production of film products for oral and peroral application according to claims 1-4 and 7, **characterised in that** contained ingredients, active ingredients and/or particles are applied by means of 3D printing by means of a 3D printer, and in the process optionally specific arrangements of the substances and three-dimensional shapes are produced.

14. Film products for oral and peroral application produced from stable, physiologically suitable cellulose fibre-based carrier matrices on a paper basis (smartFilms) according to claim 1, 2 and 4 to 12, **characterised in that** these are reduced in size and changed in their shape as intermediate products, for example by cutting or chopping, and subsequently converted into other solid (e.g. tablets, capsules or chewing gums), semi-solid (e.g. gels, ointments or chewing gums) or liquid application systems (e.g. solutions or suspensions), wherein the characteristic release properties of the film products produced from smartFilms are passed on to the respective application form together with the preservation of the crystalline state of the ingredients.

15. Process for the production of film products for oral and peroral application from stable, physiologically suitable cellulose fibre-based carrier matrix on a paper basis (smartFilm) according to claim 1-4 and 7-13, **characterised in that**, in a multi-stage loading process with intermediate drying, the laminar fibrous cellulose as a film with any active ingredients and excipients, in any desired quantity, with liquids in the form of solutions, suspensions or emulsions, in different particle sizes, at various times can be loaded, wherein liquid dispersion media can be, for example, water, ethanol, propylene glycol, liquid polyethylene glycols, glycerol, fatty oils and mixtures thereof.

16. Process for the production of film products for oral and peroral application from stable, physiologically suitable cellulose fibre-based carrier matrix on a paper basis (smartFilm) according to claim 1-4 and 7-13, **characterised in that** the loading process is carried out separately after completion of the film forming step, without thermal stressing of the ingredient or the ingredients, in particular the active ingredients, and without disturbing the film forming step.

## Revendications

1. Matrice de support stable, physiologiquement à base de fibres de cellulose, à base de papier (smartFilm), pour la fabrication de produits pelliculaires pour l'application orale et perorale, **caractérisée en ce que** celle-ci se compose de cellulose fibreuse plane à cubique sous la forme d'un matériau de papier stable constitué d'une ou plusieurs couches ayant un indice de cristallinité de 1 ≤ Krl ≤ 200 de préférence entre 18 et 70, une densité apparente en feuille de 0,10 ≤ ρ ≤ 2,00 g/cm³ de préférence entre 0,36 et 0,51 g/cm³, une capacité d'absorption d'eau de 10 ≤ M_{absorption d'eau} ≤ 2.000 % (m/m), de préférence entre 195 et 388 % (m/m), un diamètre moyen des pores de 1 ≤ dₚₒᵣₑ 200 µm, de préférence entre 21 et 57 µm, et une teneur en eau de 1,0 ≤ w ≤ 20,0 % (m/m), de préférence entre 7,5 et 9,5 % (m/m) chargés d'ingrédients, en particulier de principes actifs.

2. Matrice de support stable, physiologiquement appropriée, à base de fibres de cellulose et à base de papier (smartFilm) pour la fabrication de produits pelliculaires pour l'application orale et perorale selon la revendication 1, **caractérisée en ce que** les ingrédients, en particulier le ou les principes actifs, ont une charge relative par rapport au poids total de 0,1 ≤ β_{charge} ≤ 99,9 %, de préférence fortement chargée ≥ 70 %, incorporés dans le volume des pores et adsorbés sur la surface.

3. Matrice de support stable, physiologiquement appropriée, à base de fibres de cellulose et à base de papier (smartFilm) pour la fabrication de produits de film pour l'application orale et perorale selon la revendication 1, **caractérisée en ce qu'**elle possède une forme géométrique, par exemple une forme carrée, rectangulaire, ronde ou ovale, et possède comme forme d'application de film oral une surface de 0,1 ≤ A_{produit de film} ≤ 9 cm², de préférence 1-4 cm².

4. Matrice de support stable, physiologiquement appropriée, à base de fibres de cellulose et à base de papier (smartFilm) pour la fabrication de produits pelliculaires pour l'application orale et perorale selon les revendications 1 à 3, **caractérisée en ce que** sur celle-ci, différents ingrédients, en particulier substance(s) active(s), par exemple des analgésiques non opioïdes (par exemple acide acétylsalicylique, paracétamol, ibuprofène), des analgésiques opioïdes (par exemple tilidine, codéine, fentanyl), antibiotiques (p. ex. clarithromycine, clindamycine, minocyline), antagonistes des récepteurs bêta (p. ex. métoprolol, bisoprolol, nébivolol), antagonistes des récepteurs AT1 (p. ex. valsartan, losartan, candésartan), inhibiteurs de l'enzyme de conversion de l'angiotensine (p. ex. par ex. ramipril, captopril, énalapril), diurétiques (par ex. furosémide, hydrochlorothiazide, spironolactone), inhibiteurs des canaux calciques (par ex. nifédipine, diltiazem, vérapamil), inhibiteurs de la pompe à protons (par ex. pantoprazole, oméprazole, lansoprazole), inhibiteurs de l'HMG-CoA réductase (simvastatine, lovastatine, pravastatine), hormones thyroïdiennes (p. ex. L-thyroxine), glucocorticoïdes (p. ex. prednisolone, prednisone, dexaméthasone), antidépresseurs (p. ex. amitryptiline, miansérine, fluoxétine), neuroleptiques (p. ex. clozapine, halopéridol, perazine), benzodiazépines (p. ex. diazépam, tétrazépam, midazolam), analogues de benzodiazépines (p. ex. zaleplon, zopiclone, zolpidem), antihistaminiques (p. ex. p. ex. doxylamine, cétirizine, ranitidine, famotidine), anesthésiques locaux (p. ex. lidocaïne, tétracaïne, mépivacaïne), antiépileptiques (p. ex. carbamazépine, phénytoïne, acide valproïque), antiparkinsoniens (p. ex. lévodopa, bensérazide, carbidopa), les antidiabétiques (par exemple metformine, glibenclamide, rosiglitazone, sitagliptine, répaglinide) et les antiagrégants plaquettaires (par exemple ticlopidine, clopidogrel) sont appliqués séparément ou ensemble.

5. Matrice de support stable, physiologiquement appropriée, à base de fibres de cellulose et à base de papier (smartFilm) pour la fabrication de produits pelliculaires pour l'application orale et perorale selon les revendications 1 à 4, **caractérisée en ce que** la libération de la substance active dans l'ensemble du tube digestif, de la cavité buccale jusqu'au rectum, est prolongée, retardée, accélérée et/ou contrôlée par la charge d'adjuvants spéciaux, par exemple la gélatine, la polyvinylpyrrolidone ou l'hydroxypropylméthylcellulose.

6. Matrice de support stable, physiologiquement appropriée, à base de fibres de cellulose et à base de papier (smartFilm) selon les revendications 1 et 3, **caractérisée en ce qu'**elle n'a pas besoin de se désintégrer pour libérer les ingrédients et **en ce que** le temps de libération n'est pas prolongé par le temps de désintégration de la forme d'application, ce qui entraîne une libération immédiate par à-coups (en anglais burst release) des ingrédients due au lessivage après contact avec les liquides digestifs.

7. Matrice de support stable, physiologiquement appropriée, à base de fibres de cellulose et à base de papier (smartFilm) pour la fabrication de produits pelliculaires pour l'application orale et perorale selon les revendications 1 à 4, **caractérisée en ce que**, outre les substances actives, des colorants, des arômes, des édulcorants, des stimulants salivaires, des stabilisateurs de substances actives et des adjuvants de procédé, sans plastifiants ni stabilisateurs de support, sont présents séparément ou ensemble.

8. Matrice de support stable, physiologiquement appropriée, à base de fibres de cellulose, à base de papier (smartFilm), pour la fabrication de produits pelliculaires pour l'application orale et perorale selon les revendications 1 à 4 et 7, **caractérisée en ce qu'**elle est chargée de nanoparticules, de préférence appliquées sous forme de suspension, c'est-à-dire qu'elle contient des nanoparticules d'un diamètre inférieur à 1 mm. Nanoparticules dispersées dans un milieu de dispersion liquide aqueux ou organique, et éventuellement élimination ultérieure du milieu de dispersion, les nanoparticules étant notamment des nanoparticules de polymère, des liposomes, des cubosomes, des nanoémulsies, des nanoparticules lipidiques (solid lipid nanoparticles - SLN, nanostructured lipid carriers - NLC, smartLipids) et des micelles de tensioactifs et de polymères.

9. Matrice de support stable, physiologiquement appropriée, à base de fibres de cellulose, à base de papier (smartFilm), pour la fabrication de produits pelliculaires pour application orale et perorale selon les revendications 1 à 4 et 7, **caractérisée en ce qu'**elle est chargée de nanoparticules sous forme de nanocristaux, de préférence appliquées sous forme de nanosuspension, c'est-à-dire de nanocristaux dispersés dans un milieu de dispersion liquide aqueux ou organique, et éventuellement élimination ultérieure du milieu de dispersion.

10. Matrice de support stable, physiologiquement appropriée, à base de fibres de cellulose, à base de papier (smartFilm), pour la fabrication de produits de film pour l'application orale et perorale selon les revendications 1 à 4 et 7, **caractérisée en ce qu'**elle est chargée d'une ou de plusieurs microémulsions ou d'un ou de plusieurs systèmes auto-émulsifiants, de préférence appliqués sous forme liquide ou sous forme de préconcentré, qui forme une microémulsion ou un système auto-émulsifiant au contact de l'eau.

11. Matrice de support stable, physiologiquement appropriée, à base de fibres de cellulose, à base de papier (smartFilm), pour la fabrication de produits de film pour l'application orale et perorale selon les revendications 1 à 4 et 7, **caractérisée en ce qu'**elle est chargée de microparticules, de préférence appliquées sous forme de suspension, c'est-à-dire de microparticules dispersées dans un milieu de dispersion liquide aqueux ou organique, et éventuellement suivie d'une élimination du milieu de dispersion. Les microparticules ont une taille moyenne (diamètre 50%) de 1 µm à < 1 000 µm , les microparticules étant notamment des cristaux de principe actif, des microparticules organiques (par exemple des particules de cellulose), des particules de polymère, des liposomes, des émulsions, des microparticules de lipides et des microparticules inorganiques (par exemple de la silice).

12. Matrice de support stable, physiologiquement appropriée, à base de fibres de cellulose, à base de papier (smartFilm), pour la fabrication de produits pelliculaires pour l'application orale et perorale selon les revendications 1 à 4 et 7, **caractérisée en ce qu'**elle est chargée de microparticules poreuses, de préférence appliquées sous forme de suspension, c'est-à-dire de microparticules de la taille d'un grain de riz. Microparticules dispersées dans un milieu de dispersion liquide aqueux ou organique, ou dans du dioxyde de carbone liquide, et éventuellement élimination ultérieure du milieu de dispersion, les matériaux préférés pour les microparticules poreuses étant les celluloses, les silicates ou le carbonate de calcium, et les particules poreuses étant chargées d'un ou de plusieurs principes actifs.

13. Matrice de support stable, physiologiquement appropriée, à base de fibres de cellulose, à base de papier (smartFilm), pour la fabrication de produits pelliculaires pour l'application orale et perorale selon les revendications 1-4 et 7, **caractérisée en ce que** des ingrédients, des principes actifs et/ou des particules contenus sont appliqués au moyen d'une impression 3D au moyen d'une imprimante 3D, et des dispositions déterminées des substances et des formes tridimensionnelles sont alors produites en option.

14. Produits de film pour l'application orale et perorale fabriqués à partir de matrices de support stables, physiologiquement appropriées, à base de fibres de cellulose et à base de papier (smartFilms) selon les revendications 1, 2 et 4 à 12, **caractérisés en ce que** leur taille est réduite et leur forme modifiée en tant que produits intermédiaires, par exemple par découpage ou hachage, et qu'ils sont ensuite transformés en d'autres produits solides (par exemple en produits de nettoyage). par exemple des comprimés, des capsules ou des gommes à mâcher), des systèmes d'application semi-solides (par exemple des gels, des pommades ou des gommes à mâcher) ou liquides (par exemple des solutions ou des suspensions), les propriétés de libération caractéristiques des produits pelliculaires fabriqués à partir de smartFilms étant transmises à la forme d'application correspondante en même temps que la conservation de l'état cristallin des ingrédients.

15. Procédé de fabrication de produits pelliculaires pour l'application orale et perorale à partir d'une matrice de support stable, physiologiquement appropriée, à base de fibres de cellulose et à base de papier (smartFilm) selon les revendications 1-4 et 7-13, **caractérisé en ce que**, par un procédé de chargement en plusieurs étapes avec séchage intermédiaire, la cellulose fibreuse plane est chargée sous forme de film avec des substances actives et des adjuvants quelconques, en quantité quelconque, avec des liquides sous forme de solutions, de suspensions ou d'émulsions, dans différentes tailles de particules, à différents moments, les milieux de dispersion liquides pouvant être par exemple de l'eau, de l'éthanol, du propylèneglycol, des polyéthylèneglycols liquides, du glycérol, des huiles grasses ainsi que leurs mélanges.

16. Procédé de fabrication de produits en film pour l'administration orale et perorale à partir d'une matrice de support stable, physiologiquement appropriée, à base de fibres de cellulose et à base de papier (smartFilm) selon les revendications 1-4 et 7-13, **caractérisé en ce que** le procédé de chargement s'effectue séparément après la fin de l'étape de formation du film, sans charge thermique du ou des ingrédients, en particulier des substances actives, et sans perturbation de l'étape de formation du film.
